# EUROPÄISCHE PATENTSCHRIFT

(11) **EP 3 045 152 B1**
(45) Veröffentlichungstag und Bekanntmachung des Hinweises auf die Patenterteilung: **25.03.2020**
(21) Anmeldenummer: 16151723.0
(22) Anmeldetag: 18.01.2016
(51) Int. Cl.: A61F 5/01, A61F 5/37

(54) **VERFAHREN ZUR HERSTELLUNG EINER ORTHESE**
METHOD FOR MANUFACTURING AN ORTHOSIS
PROCÉDÉ DE FABRICATION D'UNE ORTHÈSE

(30) Priorität: 16.01.2015 DE 102015200627
(43) Veröffentlichungstag der Anmeldung: 20.07.2016
(73) Patentinhaber: Pohlig GmbH, 83278 Traunstein (DE)
(72) Erfinder: Schäfer, Michael, 83278 Traunstein (DE); Kienzle, Christian, 83064 Raubling (DE)
(74) Vertreter: Pfenning, Meinig & Partner mbB

(56) Entgegenhaltungen:
- WO-A1-2014/070621
- DE-A1-102007 005 648
- DE-A1-102008 022 952
- DE-U1-202006 011 664

## Beschreibung

Die Erfindung betrifft ein Verfahren zur Herstellung einer Orthese. Beschrieben sind auch eine Haltevorrichtung für die Herstellung einer Orthese, eine Vorrichtung zur Herstellung einer Orthese, sowie eine Orthese. Die Haltevorrichtung für die Herstellung einer Orthese zum Halten eines Körperteils in einer einstellbaren Korrekturstellung, die insbesondere Gelenkfehlstellungen korrigiert, dient als Scanvorlage, um anhand eines Abbilds des Körperteils in dieser Korrekturstellung eine Orthese zu modellieren, die zur Therapie einer Fehlstellung des Körperteils, insbesondere einer Fehlstellung des umfassten Körpergelenks, verwendet werden kann.

Orthesen dienen der Korrektur von Fehlstellungen von Körperteilen wie zum Beispiel Armen, Beinen oder dem Rumpf, insbesondere von Fehlstellungen der entsprechenden Körpergelenke. Zur Herstellung einer Orthese wurde bisher eine Kopie des betroffenen Körperteils, beispielsweise eines Unterarms, des Patienten angefertigt, indem zunächst ein Gipsabdruck des Körperteils in der gewünschten Korrekturstellung genommen wurde. Danach wurde in einem Tiefzieh- oder Laminiervorgang die Orthesenkontur auf dem Gipsmodell erzeugt, indem das Material um das Gipsmodel herumgelegt oder aus Harzen und Faserverbundstoffen gegossen wurde. Anschließend wurde die Orthese von der Gipsform in der Schleifwerkstatt entfernt. Der innere Gipskern muss bei manchen Formvarianten beispielsweise durch einen Schlagbohrer mit Meißelaufsatz herausgebrochen und die Kontur der Orthese herausgeschnitten werden. Nach dem Entfernen des inneren Kernes sowie dem groben Ausschneiden der Kontur mussten die Schnittkanten durch mehrere Schleifvorgänge nachbearbeitet werden. Dies erfolgte erst durch einen groben Schleifvorgang und dann durch finales Feinschleifen bis zur Sollkontur. Weiterhin wurden die Innenflächen zur Tragekomfortsteigerung mit einer angebrachten Fütterung versehen. Danach konnte die Orthese von einem Orthopädiemechanikermeister am Unterarm des Patienten angepasst werden. Stellen, an denen ein erhöhter Druck an der Haut vorlag oder Kanten, die in die Haut einschnitten, konnten nachträglich durch Abschleifen oder mittels lokalen Erhitzens beseitigt werden.

Dieses übliche Herstellungsverfahren weißt jedoch eine Reihe von Nachteilen auf. Durch das Gipsabdruckverfahren ist die Geometrie des Modells in der Regel überdimensioniert, wodurch beim Modellieren eine Annäherung an die originalen Maße erwirkt werden muss. Ferner kann es durch die Herstellung per Hand zu groben Fertigungsungenauigkeiten kommen, was zu einer ungleichmäßigen Auflagefläche auf dem Körperteil des Patienten führt. Hierdurch können lokale Druckstellen auf der Haut entstehen, die über einen längeren Zeitraum als besonders unangenehm empfunden werden, bis hin zu einem schmerzvollen Gefühl.

Desweiteren ist der beschriebene Herstellungsprozess aufwendig, da kaum auf Standardkomponenten zurückgegriffen werden kann. Insbesondere durch die notwendige, hohe Individualisierung der einzelnen Produkte sowie der im Vergleich zu anderen Industriesparten relativ geringen Stückzahl wird jeder Arbeitsschritt per Hand vorgenommen. Die Nachvollziehbarkeit der einzelnen Maßnahmen ist dadurch wenig objektiv und erschwert.

Da der gesamte Herstellungsprozess händisch erfolgt, kann eine hohe Oberflächengenauigkeit nicht gewährleistet werden. Insbesondere die Anfertigung der Körperteilkopie aus Gips kann deutliche Formabweichungen von mehreren Millimetern verursachen. Bei der nachfolgenden Bearbeitung dieser Gipskopie wird nach dem Erfahrungswissen der Orthopädietechnikermeister zusätzlich Material abgenommen und aufgetragen, um die Oberfläche zu glätten und Abdruckfehler zu beheben. Die ursprüngliche Kontur kann hierbei nur näherungsweise wiederhergestellt werden.

Desweiteren kann eine gleichbleibende Korrekturqualität in der Fertigung nicht gewährleistet werden. In der Regel muss bei der Anprobe der Orthese am Patienten diese nochmals nachkorrigiert werden. Auch ist am Ende des Fertigungsprozesses nicht ersichtlich, ob die zu Beginn durch den Gipsabdruck festgelegte Korrekturstellung genau erreicht werden konnte.

Hauptursache für die auftretenden Fertigungstoleranzen ist somit zum einen die Anfertigung der Körperteilkopie aus Gips, die erhebliche Formabweichungen von mehreren Millimetern erzeugt. Zum anderen sind die darauffolgenden Bearbeitungsschritte der Handschrift des jeweiligen Modellierers zuzuordnen, was sich auf die nachvollziehbare Dokumentation und Fertigungsqualität auswirkt.

In DE 10 2007 005648 A1 werden eine Vorrichtung und ein Verfahren zur Herstellung eines orthopädischen Hilfsmittels beschrieben. Um ein orthopädisches Hilfsmittel präzise anzupassen, ist es notwendig, an dem Körperteil, das mit dem orthopädischen Hilfsmittel korrespondieren soll, bereits bei der Anfertigung des orthopädischen Hilfsmittels Verschiebungen von Muskel- und anderem Gewebe zu berücksichtigen, wie sie bei der Benutzung des fertigen Hilfsmittels unter Last auftreten. Um hier den bisher üblichen langwierigen Anpassungsvorgang zu umgehen, wird eine Vorrichtung vorgeschlagen mit einer Mehrzahl von miteinander verbundenen Formteilen, die über in ihrer Position fixierbare Halter mit einem Grundgestell verbunden sind. Durch diese Formteile ist es möglich, eine räumliche Stützfläche zu erzeugen und bedarfsgerecht zu verändern, um ein Körperteil mit einer realitätsnahen Belastung und einer dementsprechend realitätsnahen Gewebeverschiebung abzustützen. Die sich ergebende Zweckform kann benutzt werden, um ein entsprechendes orthopädisches Hilfsmittel herzustellen.

In DE 10 2008 022 952 A1 werden eine nicht-invasive Extensions-, Repositions- bzw. Retentionsvorrichtung und ein entsprechendes Verfahren beschrieben. Die Vorrichtung weist einen proximalen Abschnitt mit einer Fixiereinrichtung zum temporären Fixieren des proximalen Abschnitts an einem proximal zu einer Fraktur, insbesondere in einem Sprung- und/oder einem Handgelenk, befindlichen Körperteil eines Patienten auf. Weiterhin ist ein distaler Abschnitt mit einer Immobilisationseinrichtung zum temporären Immobilisieren eines distal zu der Fraktur befindlichen Körperteils, wie eines Fußes oder einer Hand, des Patienten vorgesehen. Eine Extensionseinrichtung dient zur Extension des zuvor genannten distalen Abschnitts gegenüber dem zuvor genannten proximalen Abschnitt entlang einer Längsachse. Dabei hat die Extensions-, Repositions bzw. Retentionsvorrichtung ferner eine Ausrichtungseinrichtung zum temporären Ausrichten des distal zu der Fraktur befindlichen Körperteils, die mindestens zwei rotatorische Freiheitsgrade und/oder mindestens einen im Wesentlichen quer zur Längsachse ausgerichteten translatorischen Freiheitsgrad aufweist.

In WO 2014/070621 A1 wird eine spiralförmige Klammer beschrieben, die eine innere Oberfläche aufweist, die einer digitalen Darstellung einer verletzten Extremität entspricht. Die spiralförmige Klammer kann spiralförmig um eine Gliedmaße gewickelt werden. Der Körper der spiralförmigen Klammer kann eine einstückig ausgebildet sein und Öffnungen zum Bereitstellen einer Luftzirkulation zu der verletzten Extremität aufweisen.

In DE 20 2006 011 664 U1 wird eine Mittelhand-Daumen-Orthese zur Rückstellung der Mittelhand mit einer an Teilbereiche der Hand anlegbaren elastischen Schiene und mindestens einem Verschlußelement beschrieben. Die Schiene ist dabei in Längserstreckung offen und symmetrisch ausgebildet und weist mindestens ein Verschlußelement im daumenseitigen Bereich und mindestens ein Verschlußelement im handgelenkseitigen Bereich auf.

Es ist daher eine Aufgabe der vorliegenden Erfindung, die oben beschriebenen Probleme des Standes der Technik zu lösen und ein Verfahren zu entwickeln, das aus einem Scanmodell eine Orthese aus definierten Kenngrößen konstruiert, simuliert und hergestellt werden kann und welche durch diese Methodik zu einer höheren Versorgungsqualität und Präzision sowie einer strukturierten Arbeitsweise und Nachvollziehbarkeit in allen Fertigungsstufen führen.

Die Aufgabe wird durch ein Verfahren nach Anspruch 1 gelöst. Vorteilhafte Weiterbildungen des erfindungsgemäßen Verfahrens sind in den abhängigen Ansprüchen beschrieben.

Die Haltevorrichtung für die Herstellung einer Orthese zum Halten eines Körperteils in einer einstellbaren Korrekturstellung als Scanvorlage weist eine Mehrzahl von Haltebereichen und eine oder mehrere Korrekturkraftstellvorrichtungen auf, wobei mindestens eine der Korrekturkraftstellvorrichtungen mit mindestens einem der Haltebereiche verbunden ist. Mittels der Korrekturkraftstellvorrichtungen sind Korrekturkräfte über die mit den Korrekturkraftstellvorrichtungen verbundenen Haltebereiche auf den Körperteil ausübbar, und hierdurch der Körperteil in eine Korrekturstellung bringbar.

Die mittels der Korrekturkraftstellvorrichtungen ausübbaren Korrekturkräfte weisen bevorzugt mindestens eine senkrecht zur Oberfläche des Körperteils gerichtete Kraftkomponente auf. Insbesondere sind mittels der Korrekturkraftstellvorrichtungen Korrekturkräfte über die mit den Korrekturkraftstellvorrichtungen verbundenen Haltebereiche auf ein von dem Körperteil umfassten Körpergelenk ausübbar, und hierdurch das Körpergelenk in eine Korrekturstellung bringbar.

In einer besonders bevorzugten Ausgestaltung kann die Haltevorrichtung einen oder mehrere Kraftsensoren zum Messen der auf den Körperteil ausgeübten Korrekturkräfte aufweisen. Übt die Haltevorrichtung beispielsweise auf einen Körperteilbereich eine Korrekturkraft in eine bestimmte Richtung aus, so wirkt der Körperteilbereich dieser Korrekturkraft mit einer Gegenkraft in eine entgegengesetzte Richtung entgegen. Diese Gegenkraft wirkt später ebenfalls auf die an den Körperteil angelegte Orthese. Somit entspricht eine auf einen Körperteilbereich ausgeübte Korrekturkraft auch einer von diesem Körperteilbereich auf die später an den Körperteil angelegte Orthese ausgeübten Gegenkraft. Anhand der durch den einen oder die mehreren Kraftsensoren gemessenen Korrekturkräfte kann daher eine Belastungsverteilung entlang der späteren Orthese vorausberechnet werden. Die Haltevorrichtung ermöglicht es, auf einen Gipsabdruck sowie auf ein Gipsmodell des sich in einer Korrekturstellung befindlichen Körperteils zur Herstellung einer Orthese zu verzichten, da der Körperteil direkt in der Haltevorrichtung gescannt werden kann und so ein digitales Abbild des Körperteils in der gewählten Korrekturstellung erstellt werden kann. Anhand dieses digitalen Abbildes des Körperteils kann dann über eine vordefinierte Modelltechnik ein digitales Orthesenmodell erzeugt werden, aufgrund dessen später die Orthese präzise mit hoher Oberflächengenauigkeit und gleichbleibender Korrekturqualität gefertigt werden kann.
Durch die optionalen Kraftsensoren ermöglicht die erfindungsgemäße Haltevorrichtung eine spätere Belastungsverteilung der Orthese zu berechnen, und die Daten über die Belastungsverteilung zur Optimierung der Materialdichte der Orthese zu verwenden. Hierdurch ist es möglich eine individuelle Orthese zu fertigen, die nur eine Mindestmenge an Material und nur ein Mindestgewicht aufweist. Dies kann wiederum zu einem höheren Tragekomfort für den Patienten beitragen, da die Orthese so leicht wie möglich und luftdurchlässig konstruiert werden kann. Das im Stand der Technik beschriebene Problem der Überdimensionierung der Orthese wird somit ebenso beseitigt.

Bevorzugt weist die Haltevorrichtung mindestens drei Haltebereiche auf. Drei Haltebereiche ermöglichen eine präzise Halterung des Körperteils in einer beliebigen Korrekturstellung.

Weiter ist es vorteilhaft, wenn jeder Haltebereich ein, zwei oder mehrere Halteelemente zum Fixieren des Körperteils in jeweils mindestens einer Raumrichtung und/oder zum Übertragen der mittels der Korrekturkraftstellvorrichtungen ausgeübten Korrekturkräfte an den Körperteil aufweist. Durch die Halteelemente können Korrekturkräfte in einer oder in mehreren gewünschten Richtungen präzise auf den Körperteil übertragen werden.

Ferner ist es vorteilhaft, wenn die Halteelemente weniger als 30 %, vorteilhafterweise weniger als 20 %, besonders bevorzugt weniger als 10% der Oberfläche des zu haltenden Körperteils überdecken. Dies ermöglicht ein besonders detailliertes digitales Abbild des Körperteils in der gewünschten Korrekturstellung, wenn das Körperteil mit der Haltevorrichtung gescannt wird.

Mindestens einer der Kraftsensoren kann in eines der Halteelemente integriert sein. Auch eine Mehrzahl der oder alle Kraftsensoren können in einige der oder alle Halteelemente integriert sein. Der Vorteil der Integration der Kraftsensoren in die Halteelemente besteht darin, dass die auf den Körperteil übertragene Korrekturkraft direkt dort gemessen werden kann, wo die Korrekturkraft übertragen wird. Hierdurch kann eine spätere Belastungsverteilung entlang der Orthese präziser bestimmt werden.

Die Kraftsensoren können Drucksensoren oder Druckmessgeräte umfassen. Die gemessenen Korrekturkräfte sind dann im Wesentlichen Druckkräfte, die von den Halteelementen auf den Körperteil ausgeübt werden.

Die Position der Kraftsensoren an der Haltevorrichtung ist abhängig von der Fehlstellung des von der Haltevorrichtung gehaltenen Körperteils. Vorteilhaft ist es, wenn die Kraftsensoren in Bereichen mit großer Hebelwirkung, also maximal entfernt von einem Umlenkpunkt, und am Umlenkpunkt selbst angeordnet sind. Dies ermöglicht eine exakte Messung der auf den Körperteil wirkenden Korrekturkräfte.

Mindestens eine der Korrekturkraftstellvorrichtungen kann ferner eine längenverstellbare Stange, eine Teleskopstange, eine Linearführung, einen Linearzylinder, jeweils zwei Schenkel deren Winkelstellung justierbar ist, einen mehrgelenkigen Arm und/oder ein justierbares Scherengelenk aufweisen. Besonders vorteilhaft ist es, wenn die mindestens eine Korrekturkraftstellvorrichtung einen drei-gelenkigen Arm aufweist, der ein mittleres und zwei äußere Gelenke aufweist, wobei das mittlere Gelenk ein Scharniergelenk ist und die äußeren Gelenke Kugelgelenke sind, und wobei mindestens eines der äußeren Gelenke mit einem der Haltebereiche verbunden ist, und durch Variation einer Stellung der äußeren und des mittleren Gelenks Korrekturkräfte ausübbar sind. Dies ermöglicht eine schnelle, beliebige und stufenlose Einstellung einer Korrekturstellung.

Desweiteren ist es vorteilhaft, wenn die Korrekturkräfte je nach zu erreichender Korrekturstellung variierbar sind. Hierdurch ist es möglich, die Haltevorrichtung derart einzustellen, dass der Körperteil in eine beliebige vom Orthopädietechniker bestimmte Korrekturstellung gebracht werden kann. Dabei kann sowohl der Betrag als auch die Richtung der Korrekturkraft variiert werden.

In einer bevorzugten Ausgestaltung können die Halteelemente eines Haltebereichs oder die Halteelemente verschiedener Haltebereiche durch eine Anpassungsstellvorrichtung zur genauen Anpassung der Haltevorrichtung an eine Größe des Körperteils miteinander verbunden sein.

Ferner kann die Haltevorrichtung derart gestaltet sein, dass die Vorrichtung mit Halteelementen verschiedener Formen und Größen, gegebenenfalls auch individuell anpassbaren Halteelementen, kompatibel ist und die Halteelemente austauschbar sind. Auf diese Weise kann die Haltevorrichtung sowohl für Kinder als auch für Erwachsene oder für verschiedenartige Körperteile verwendet werden und entsprechend optimal angepasst werden.

Ferner können auch zwei Haltebereiche über eine Korrekturkraftstellvorrichtung miteinander verbunden sein, mittels welcher eine Korrekturkraft auf einen ersten Haltebereich relativ zu einem zweiten Haltebereich ausübbar ist. Hierdurch kann beispielsweise auf die Verwendung eines Grundgestells verzichtet werden, an dem die Korrekturkraftstellvorrichtungen befestigt sind und relativ zu dem die Korrekturkräfte auf den Körperteil ausgeübt werden. Dies ermöglicht eine uneingeschränktere Sicht auf den gesamten Körperteil beim Scannen und es müssen später weniger Störbereiche, also mitgescannte Bereiche der Haltevorrichtung, vom Scan des Körperteils entfernt werden.

Weiterhin ist eine Haltevorrichtung ohne Grundgestell vorteilhaft, da eine Scanvorrichtung zum Scannen des von der Haltevorrichtung gehaltenen Körperteils einfacher herstellbar ist. Ferner ist eine Haltevorrichtung ohne Grundgestell mobiler. Die Haltevorrichtung ohne Grundgestell ähnelt der endgültigen Orthese, d.h. die Korrekturkräfte werden in ähnlicher Weise auf das Körperteil übertragen. Hierdurch können sich die zwischen dem Körperteil und der Haltevorrichtung wirkenden Kräfte neutralisieren, wohingegen ein Grundgestell Kräfte aufnimmt und die zwischen dem Körperteil und der Haltevorrichtung wirkenden Kräfte verändert. Eine Haltevorrichtung ohne Grundgestell kann auch Funktionsprüfungen des mit der zukünftigen Orthese zu behandelnden Körperteils, wie z.B. Greiffunktionstests, ermöglichen.

In einer weiteren bevorzugten Ausführungsform weist die Haltevorrichtung ein Grundgestell auf, welches einen Aufnahmebereich für den Körperteil definiert und an dem die Korrekturkraftstellvorrichtungen befestigt sind, welche sich an dem Grundgestell abstützen und geeignet sind Korrekturkräfte relativ zum Grundgestell über die mit den Korrekturkraftstellvorrichtungen verbundenen Haltebereiche auf den Körperteil auszuüben. Durch die Verwendung eines Grundgestells wird ermöglicht, voneinander unabhängige Korrekturkräfte in verschiedenen Korrekturrichtungen auf den Körperteil auszuüben. Hierdurch wird die Anzahl der Korrekturstellungen, welche mit der Haltevorrichtung erreicht werden können, weiter erhöht. Ferner können die Korrekturkräfte und damit auch die möglichen Korrekturstellungen präziser eingestellt werden.

Das Grundgestell kann auch lediglich in einer Schiene, einer Stange und/oder einer Teleskopstange bestehen. Je weniger Komponenten der Haltevorrichtung die freie Sicht auf das Körperteil beim Scannen behindern, um so genauer kann das digitale Abbild erzeugt werden.

Das Grundgestell kann ferner parallel oder in einem Winkel zueinander verlaufende Schienen umfassen, welche in einer zu korrigierenden Richtung oder in einer radialen Richtung den Aufnahmebereich definieren und an welchen die Korrekturkraftstellvorrichtungen befestigt sind, wobei ein gemeinsamer Querschnitt durch die Schienen senkrecht zur Längsausdehnung der Schienen ein regelmäßiges oder unregelmäßiges n-Eck bildet, und wobei zueinander benachbarte Schienen durch mindestens ein Verbindungselement miteinander starr verbunden sind, und wobei n ≥ 3 ist.

Das mindestens eine Verbindungselement kann dabei eine einstellbare Länge und/oder Form aufweisen, wodurch der Abstand der Schienen zueinander und/oder die Geometrie des gemeinsamen Querschnitts variierbar sind. Hierdurch lässt sich die Haltevorrichtung individuell an den Körperteil des Patienten anpassen und zur Erreichung einer bestimmten Korrekturstellung genauer einstellen.

Weiter können Positionen der Korrekturkraftstellvorrichtungen auf den Schienen mittels Schnellspannern, Stellschrauben, Lochschienen und/oder Rastelementen einstellbar sein. Auch hierdurch lässt sich die Haltevorrichtung weiter individuell an den Körperteil des Patienten anpassen bzw. zur Einstellung einer gewünschten Korrekturstellung weiter anpassen. Dabei erfolgt die Anpassung schnell, einfach und im Wesentlichen werkzeugfrei.

Vorteilhafterweise weist mindestens eine der Korrekturkraftstellvorrichtungen eine längenverstellbare Stange, insbesondere eine Gewindestange, auf und istderart an den Schienen befestigt, dass eine Längsachse der längenverstellbaren Stange weitgehend senkrecht zu den Schienen ausgerichtet ist und durch eine Variation der Länge der Stange Korrekturkräfte auf den Körperteil variierbar sind. Auf diese Weise können die Korrekturkräfte effizient auf den Körperteil übertragen werden.

Weiterhin ist es vorteilhaft, wenn die Korrekturkraftstellvorrichtungen mittels eines Schnellspanners oder eines Schnellverschlusses zur Ausübung einer bestimmten Korrekturkraft arretierbar sind. So können die gewünschten Korrekturkräfte und damit auch die gewünschte Korrekturstellung schnell und einfach eingestellt werden.

Ferner ist es bevorzugt, wenn höchstens zwei Korrekturkraftstellvorrichtungen an derselben Schiene befestigt sind. Hierdurch wird eine stabile, präzise und effiziente Einstellung der gewünschten Korrekturstellung ermöglicht. Ferner ist bei einer derartigen Anordnung der Korrekturkraftstellvorrichtungen eine größere Zahl verschiedener Korrekturstellungen möglich.

Die Schienen können formstabile Materialien, insbesondere Metall oder Kunststoff, insbesondere thermoplastische, duroplastische und/oder elastomere Kunststoffe aufweisen oder daraus bestehen.

Die Halteelemente können formstabile Materialien, insbesondere Metall oder Kunststoff, insbesondere thermoplastische, duroplastische und/oder elastomere Kunststofffe, oder Glas aufweisen oder daraus bestehen.

Die Halteelemente können ferner eine Körperkontaktseite aufweisen, mit welcher sie den Körperteil mechanisch kontaktieren. Weiter ist es vorteilhaft, wenn die Halteelemente an ihrer Körperteilkontaktseite gepolstert sind. Hierdurch wird vermieden das eventuelle Kanten der Halteelemente zu Druckstellen an dem Körperteil führen, wenn es sich in der Korrekturstellung in der Haltevorrichtung befindet.

Offenbart ist auch eine Vorrichtung zum Erfassen von Patientendaten, wobei Patientendaten Geometriedaten zur Beschreibung einer Geometrie des Körperteils umfassen. Die Vorrichtung weist eine oben beschriebene Haltevorrichtung, ein Scangerät zum Scannen der Geometrie des von der Haltevorrichtung gehaltenen Körperteils und Erzeugen der Geometriedaten und ein Speichergerät zum Speichern der Patientendaten auf. Vorteilhafterweise umfassen die Patientendaten auch Kraftmessdaten zur Beschreibung der von den Korrekturkraftstellvorrichtungen auf den Körperteil ausgeübten Korrekturkräfte.
Durch die Verwendung von Patientendaten kann die Herstellung einer Orthese automatisiert durchgeführt werden, wodurch die Orthese präziser herstellbar ist und die Herstellung in einer nachhaltigeren Weise dokumentiert werden kann.

Offenbart ist auch eine Vorrichtung zur Herstellung einer Orthese mit einer oben beschriebenen Vorrichtung zum Erfassen von Patientendaten, einer Datenverarbeitungseinrichtung zum Erzeugen eines digitalen Orthesenmodells und zum Erzeugen eines angepassten Orthesenmodells der Orthese, in dem das digitale Orthesenmodell basierend auf den Patientendaten an dem Körperteil individuell angepasst wird, und einer Fertigungsvorrichtung zur Fertigung der Orthese basierend auf dem angepassten Orthesenmodell.

Mittels der Vorrichtung zur Herstellung einer Orthese wird eine möglichst konturgenaue Gestaltung der Geometrie der Orthese ermöglicht.

Vorzugsweise umfasst die Fertigungsvorrichtung eine Vorrichtung zur additiven Fertigung, insbesondere eine Lasersinteranlage.

Offenbart ist auch ein Verfahren zum Erfassen von Patientendaten, wobei Patientendaten mittels einer oben beschriebenen Vorrichtung zum Erfassen von Patientendaten erfasst werden. Das Verfahren zum Erfassen von Patientendaten kann insbesondere die Schritte umfassen, dass der Körperteil in einer eingestellten Korrekturstellung in der Haltevorrichtung gehalten wird, die Geometrie des Körperteils von dem Scangerät gescannt wird, die Geometriedaten erzeugt werden, gegebenenfalls die Korrekturkräfte in der Haltevorrichtung gemessen und die Kraftmessdaten erzeugt werden, und die Patientendaten in dem Speichergerät gespeichert werden.

Erfindungsgemäß ist ein Verfahren zur Herstellung einer Orthese, wobei eine Orthese mittels einer oben beschriebenen Vorrichtung zur Herstellung der Orthese hergestellt wird. Das Verfahren zur Herstellung der Orthese kann insbesondere die Schritte umfassen, dass mit der oben beschriebenen Vorrichtung zum Erfassen von Patientendaten, Patientendaten erfasst werden, ein digitales Orthesenmodell der Orthese für den Körperteil erzeugt wird, das digitale Orthesenmodell basierend auf den Patientendaten an den Körperteil individuell angepasst wird und so ein angepasstes Orthesenmodell der Orthese erzeugt wird, wobei gegebenenfalls mittels der Kraftmessdaten eine Belastungsverteilung aufgrund von vom Körperteil ausgeübten Gegenkräften in Reaktion auf die Korrekturkräfte entlang des digitalen Orthesenmodells berechnet und eine Struktur der Orthese in dem digitalen Orthesenmodell entsprechend der berechneten Belastungsverteilung optimiert wird, und die Orthese basierend auf dem angepassten Orthesenmodell gefertigt wird.

In einer vorteilhaften Ausgestaltung des Verfahrens zur Herstellung einer Orthese wird die Orthese mittels eines additiven Fertigungsverfahrens basierend auf dem angepassten Orthesenmodell gefertigt.

Offenbart ist auch eine Orthese zur Korrektur einer Fehlstellung eines Körperteils, wobei die Orthese mittels eines oben beschriebenen Verfahrens zur Herstellung einer Orthese hergestellt wird. Die Orthese ermöglicht ferner ein Entlasten und Betten eines Körperteils bzw. eines Körpergelenks, wobei sie dynamisch und fixierend ist.

Ferner kann die so hergestellte Orthese bereichsweise oder vollständig eine perforierte Struktur und/oder eine Gitterstruktur aufweisen, die zumindest bereichsweise entsprechend der berechneten und/oder gewünschten Belastungsverteilung, insbesondere für flexible und/oder steife Wände, angepasst ist. Die Gitterstruktur kann offen oder geschlossen ausgebildet sein. Bei der offenen Gitterstruktur weist die Gitterstruktur Gitteröffnungen auf, die von einem Gitter begrenzt werden. Bei der geschlossenen Gitterstruktur sind die Gitteröffnungen durch eine Materialschicht verschlossen sind, welche eine geringere oder ein gleiche Materialstärke wie das Gitter aufweist.

Durch die Verwendung einer Gitterstruktur kann die Orthese leichter gefertigt werden bei gleicher Stabilität. Zusätzlich ermöglicht die offene Gitterstruktur eine gute Belüftung eines von der Orthese aufgenommenen Körperteils. Dies erhöht den Tragekomfort der Orthese. Gleichzeitig eröffnet die Verwendung einer offenen Gitterstruktur vielfältige Designoptionen, wodurch wiederum die Akzeptanz der Orthese durch den Patienten verbessert wird.

Bei der Gitterstruktur kann es sich um ein zweidimensionales Gitter handeln, dass sich in tangentialer Richtung, also parallel zur Oberfläche eines Körperteils erstreckt, wenn die Orthese an dieses angelegt ist. Ferner kann es vorteilhaft sein, wenn die Orthese bereichsweise oder vollständig eine Wandung mit einer oder mehreren Lagen, insbesondere zwei oder drei Lagen, aufweist und die Anzahl der Lagen entlang der Orthese variiert oder konstant ist. In diesem Fall kann es sich bei der Wandung beispielsweise um eine dreidimensionale Gitterstruktur handeln, welche in radialer Richtung, also in einer Richtung, die senkrecht zur Oberfläche eines Körperteils verläuft, wenn an dieses die Orthese angelegt ist, mindestens eine weitere Gitterebene (zweite Lage) aufweist, die sich ebenfalls in tangentialer Richtung erstreckt. Es ist auch denkbar, dass die Gitterstruktur in verschiedenen Bereichen der Orthese eine verschiedene Anzahl an in radialer Richtung angeordneten, in tangentialer Richtung verlaufenden Gitterebenen (Lagen) aufweist . Ferner kann die Anzahl dieser Gitterebenen (Lagen) in tangentialer Richtung der Orthese variieren. Vorzugsweise weist die Wandung der Orthese in stark belasteten Bereichen der Orthese eine größere Anzahl an Lagen auf als in weniger belasteten Bereich der Orthese. Auf diese Weise können in der Orthese weiche Areale ausgebildet werden, um knöcherne Bewegungen bettend zu führen.

Im Folgenden werden einige Beispiele einer Haltevorrichtung, einem Verfahren zum Erfassen von Patientendaten und eines erfindungsgemäßen Verfahrens zur Herstellung einer Orthese anhand von Figuren ausführlicher beschrieben. Dabei werden verschiedene erfindungswesentliche oder vorteilhafte Elemente im Rahmen dieser Beispiele genannt, wobei auch einzelne dieser Elemente als solche zur Weiterbildung der Erfindung - auch herausgelöst aus dem Kontext der Beispiele - verwendet werden können. Ferner entsprechen in den Figuren gezeigte gleiche oder ähnliche Elemente gleichen oder ähnliche Bezugszeichen.

Es zeigen:
- Fig. 1a: ein erstes Ausführungsbeispiel einer Haltevorrichtung, die einen Unterarm in einer eingestellten Korrekturstellung hält, in einer Draufsicht,
- Fig. 1b: ein weiteres Ausführungsbeispiel einer Haltevorrichtung die einen Unterarm in einer eingestellten Korrekturstellung hält, in einer Draufsicht,
- Fig. 2: die Haltevorrichtung aus Fig. 1b, die einen Unterarm in einer eingestellten Korrekturstellung hält, in einer Seitenansicht,
- Fig. 3: die Haltevorrichtung aus Fig. 1b, die einen Unterarm in einer eingestellten Korrekturstellung hält, in einer Unteransicht.
- Fig. 4: ein weiteres Ausführungsbeispiel einer Haltevorrichtung in einer Draufsicht,
- Fig. 5: die Haltevorrichtung aus Fig. 4, die einen Unterarm in einer eingestellten Korrekturstellung hält, in einer Seitenansicht,
- Fig. 6: die Haltevorrichtung aus Fig. 4, die einen Unterarm in einer eingestellten Korrekturstellung hält, in einer Draufsicht,
- Fig. 7: ein weiteres Ausführungsbeispiel einer Haltevorrichtung für einen Unterarm in einer Perspektivansicht,
- Fig. 8: die Haltevorrichtung aus Fig. 7, die ein Unterarmmodell hält, in einer perspektivischen Ansicht,
- Fig. 9: ein weiteres Ausführungsbeispiel einer Haltevorrichtung, die einen Unterarm in einer eingestellten Korrekturstellung hält, in einer Perspektivansicht,
- Fig. 9a: ein weiteres Ausführungsbeispiel einer Haltevorrichtung, die ebenfalls einen Unterarm in einer eingestellten Korrekturstellung hält, in einer Perspektivansicht,
- Fig. 9b: eine Weiterentwicklung der Haltevorrichtung aus Fig. 9a als ein weiteres Ausführungsbeispiel einer Haltevorrichtung in einer Perspektivansicht,
- Fig. 9c: die Haltevorrichtung aus Fig. 9b in einer weiteren Perspektivansicht,
- Fig. 10: ein Prozessdiagramm eines erfindungsgemäßen Verfahrens zur Herstellung einer Unterarmorthese,
- Fig. 11: ein erfindungsgemäß erzeugtes Orthesenmodell für einen Unterarm in einer Perspektivansicht,
- Fig. 12: eine Wandung einer Orthese mit einer Gitterstruktur in einer Perspektivansicht und
- Fig. 13: die Wandung aus Fig. 12 in einer Draufsicht.

Im Weiteren werden die folgenden Bezugszeichen verwendet:
- 1: Haltevorrichtung
- 2: Unterarm
- 3a, 3b, 3c: Haltebereich
- 4a, 4b, 4c: Halteelement
- 4a', 4b', 4c': Halteelement
- 5a, 5b, 5c: Korrekturkraftstellvorrichtung
- 5a', 5b', 5c': Korrekturkraftstellvorrichtung
- 6a, 6b, 6c, 6d, 6e: Anpassungsstellvorrichtung
- 7: Mittelhand-Daumenführung
- 50b: zylinderförmiges Gehäuse
- 51b, 52b: Justierelement
- 53b: Stellhebel
- 60a-d, 50a: zylinderförmiges Gehäuse
- 61a-d, 51a: Stange
- 62a-d, 52a: Stellrad
- 53a: Stellrad
- 54a: Führungsschiene
- 8: Grundgestell
- 8a, 8b, 8c: Elemente der Teleskopstange
- 63, 63',64, 64': Kugelgelenk
- 65, 65': Scharniergelenk
- 66, 66': Schnellverschluss
- 67, 67': Gelenkarme
- 9: Schienen
- 10, 10': Verbindungselement
- 11: Drucksensor
- 11a: Anzeige
- 12: Aussparung
- 13: Standschienen
- 14, 15: Stützschienen
- 16: Standfüße
- 70: Klemmblock
- 71, 72: Drehhebel
- 73: Stangen
- 74: Schnellspanner
- 110: UASO-Modell
- 111: erster Stützbereich
- 111a: Daumenführung
- 112: zweiter Stützbereich
- 113: dritter Stützbereich
- 114: Gitterstruktur
- 115: äußere Gitterebene
- 116: innere Gitterebene
- 117: Gitterstege

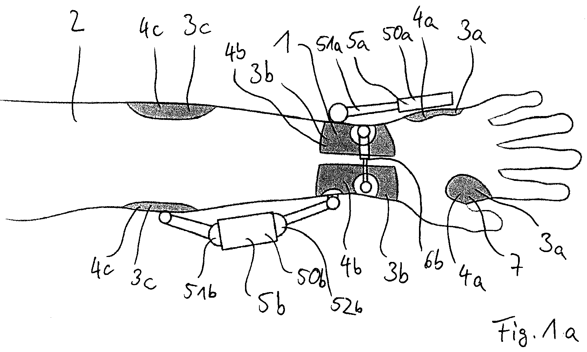

Zunächst wird ein erstes Ausführungsbeispiel einer Haltevorrichtung zum Halten eines Körperteils in einer einstellbaren Korrekturstellung mit Bezug auf Fig. 1a beschrieben. Fig. 1a zeigt eine Haltevorrichtung 1 zum Halten eines Unterarms 2 in einer eingestellten Korrekturstellung. Die Haltevorrichtung 1 weist drei Haltebereiche 3a, 3b, 3c auf, wobei ein erster Haltebereich 3a zur Aufnahme einer Handfläche, ein zweiter Haltebereich 3b zur Aufnahme eines Handgelenk-nahen Bereichs eines Unterarms und ein dritter Haltebereich 3c zur Aufnahme einer Unterseite eines Unterarms in etwa mittig zwischen dem Ellbogengelenk und dem Handgelenk ausgebildet ist. In Fig. 1a ist die Haltevorrichtung 1 sowie der Unterarm von oben zu sehen. Die drei Haltebereiche 3a, 3b, 3c ermöglichen eine so genannte Dreipunktauflage (auch Drei-Punkte-Prinzip genannt), in der das Handgelenk durch den Haltebereich 3b in eine Richtung geführt wird, und durch zwei weitere Haltebereiche 3a und 3c in eine entgegengesetzte Richtung eine Korrekturkraft aufgebracht wird.

Ferner weist der Haltebereich 3a im Bereich der Mittelhand-Daumen-Region eine Mittelhand-Daumenführung 7 auf, welche handinnenflächenseitig angebracht ist und die Lage des Daumens stützen und korrigieren soll. Der Haltebereich 3a ist im Wesentlichen in zwei Halteelemente 4a geteilt, welche durch eine Anpassungsstellvorrichtung 6a (hier nicht gezeigt) miteinander verbunden sind. Der Haltebereich 3b ist im Wesentlichen in zwei Halteelemente 4b geteilt, welche durch eine Anpassungsstellvorrichtung 6b abstandsvariabel miteinander verbunden sind. Der Haltebereich 3c ist im Wesentlichen in zwei Halteelemente 4c geteilt, welche abstandsvariabel durch eine Anpassungsstellvorrichtung 6c (hier nicht gezeigt) miteinander verbunden sind. Mithilfe der Anpassungsstellvorrichtungen 6a, 6b, 6c kann die Haltevorrichtung an eine Größe, insbesondere eine Dicke des Unterarms individuell angepasst werden. Bei den Stellvorrichtungen 6a, 6b, 6c handelt es sich um feststellbare Teleskopstangen, welche zur Änderung einer Weite der Haltebereiche verkürzt oder verlängert werden können.

Der Haltebereich 3a umgreift dabei die Hand in einer Umfangsrichtung von der Handinnenfläche aus und ist über den Handrücken weitgehend geöffnet, so dass eine Abstützung an der Handinnenfläche, den Handseitenflächen und an den Seitenbereichen des Handrückens erfolgt. Der Haltebereich 3b umgreift das Handgelenk von einer Oberseite des Unterarms 2 aus über die Seitenflächen des Handgelenks bis zu den Seitenbereichen der Handgelenksunterseite, so dass eine Abstützung des Handgelenks an der Oberseite, an den Seiten und in den Seitenbereichen der Unterseite des Handgelenks erfolgt. Der Haltebereich 3c umgreift den Unterarm 2 von einer Unterseite des Unterarms 2 aus über die Seitenflächen des Unterarms 2 bis zu den Seitenbereichen der Oberseite des Unterarms 2, so dass eine Abstützung des Unterarms 2 entlang der Unterseite des Unterarms 2, der Seitenflächen des Unterarms 2 und der Seitenbereiche der Oberseite des Unterarms 2 erfolgt. Desweiteren weist die Haltevorrichtung 1 zwei Korrekturkraftstellvorrichtungen 5a und 5b zur Ausübung von Korrekturkräften auf. Die Korrekturkraftstellvorrichtung 5b ist mit den Haltebereichen 3b und 3c an der zum Körper hin gelegenen Innenfläche des Unterarms 2 verbunden. Die Korrekturkraftstellvorrichtung 5b weist ein zylinderförmiges Gehäuse 50b auf, sowie an den offenen Seiten des zylinderförmigen Gehäuses 50b kugelgelenkartig gelagerte Justierelemente 51b und 52b. Das Justierelement 51b ist mit dem Haltebereich 3c kugelgelenkartig verbunden, während das Justierelement 52b mit dem Haltebereich 3b kugelgelenkartig verbunden ist. Jede der kugelgelenkartigen Verbindungen zwischen den Haltebereichen 3b und 3c und den Justierelementen 51b und 52b, sowie zwischen den Justierelementen 51b und 52 b und dem Gehäuse 50b können derart festgestellt werden, dass der Unterarm 2 in eine vorbestimmte feste Korrekturstellung gebracht werden kann.

Die Korrekturkraftstellvorrichtung 5a verbindet die Haltebereiche 3a und 3b an einer dem Körper abgewandten Außenseite des Unterarms 2 miteinander und weist ein zylinderförmiges Gehäuse 50a auf, welches mit dem Haltebereich 3a an der Handaußenseite fest verbunden ist. In dem zylinderförmigen Gehäuse 50a ist eine Stange 51a derart gelagert, dass diese Stange 51a in das zylinderförmige Gehäuse 50a einschiebbar oder aus dem zylinderförmigen Gehäuse 50a herausschiebbar ist. Die Stange 51a und das zylinderförmige Gehäuse 50a bilden somit eine Teleskopstange, welche in der Länge variierbar ist. Die Stange 51a ist darüber hinaus an einer Außenseite des Handgelenks mit dem Haltebereich 3b kugelgelenkartig verbunden, so dass sich eine in der Haltevorrichtung 1 befindliche Hand sowohl nach außen oder innen als auch nach oben oder unten bewegen lässt. Sowohl die kugelgelenkartige Verbindung zwischen der Stange 51a und dem Haltebereich 3b als auch die teleskopstangenartige Verbindung zwischen der Stange 51a und dem zylinderförmigen Gehäuse 50a sind feststellbar, so dass sich die Stellung der Hand relativ zum Unterarm 2 in eine vorbestimmte Korrekturstellung bringen lässt.

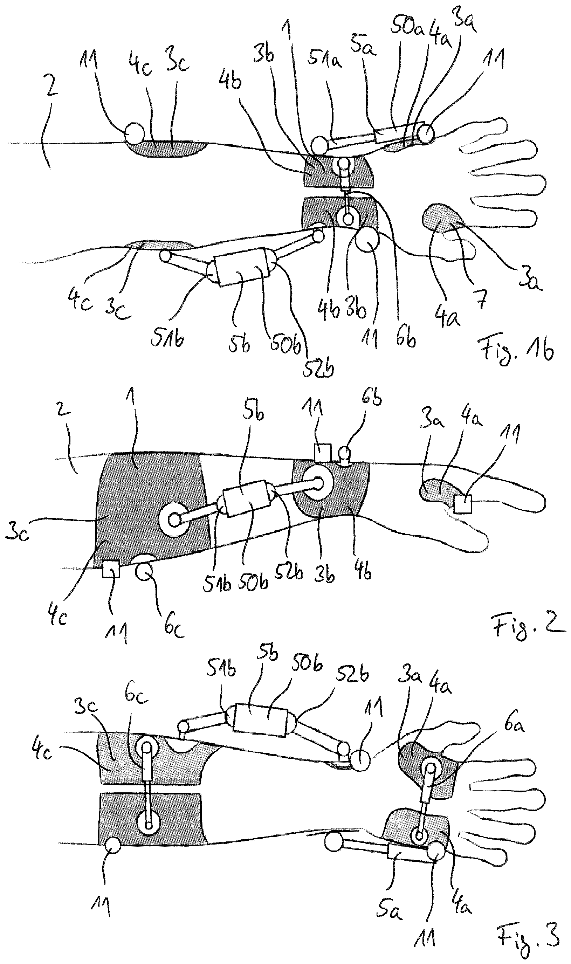

Fig. 1b zeigt ein weiteres Ausführungsbeispiel, welches sich von dem in Fig. 1a gezeigten Ausführungsbeispiel nur durch an der Haltevorrichtung 1 zusätzlich befestigte Drucksensoren 11 unterscheidet. Am dem Körper abgewandten, äußeren Halteelement 4c ist ein Drucksensor 11 zur Messung von Korrekturkräften angeordnet, welche durch die Korrekturkraftstellvorrichtung 5b vom dem Körper abgewandten, äußeren Halteelement 4c auf den Unterarm ausgeübt werden. Ferner ist ein weiterer Drucksensor 11 am dem Körper zugewandten Halteelement 4b zur Messung von Korrekturkräften angeordnet, welche durch die Korrekturkraftstellvorrichtungen 5a, 5b vom dem Körper zugewandten Halteelement 4b auf den Unterarm ausgeübt werden. Ein weiterer Drucksensor 11 ist am dem Körper abgewandten Halteelement 4a zur Messung von Korrekturkräften angeordnet, welche durch die Korrekturkraftstellvorrichtung 5a vom dem Körper abgewandten Halteelement 4a ausgeübt werden.

Fig. 2 zeigt die Haltevorrichtung aus Fig. 1b, in welcher sich der Unterarm 2 in einer vorbestimmten Korrekturstellung befindet, in einer Ansicht auf die Innenseite des Unterarms 2. Zusätzlich zu der Anpassungsstellvorrichtung 6b des Haltebereichs 3b weist die Haltevorrichtung 1 eine Anpassungsvorrichtung 6c im Haltbereich 3c auf. Die Anpassungstellvorrichtung 6c ist auf der Unterseite der Haltevorrichtung 1 angeordnet und ermöglicht eine Weitenverstellung des Haltebereichs 3c, wobei sich der Abstand der beiden Halteelemente 4c mittels der Anpassungsstellvorrichtung 6c vergrößern oder verkleinern lässt. Die Anpassungstellvorrichtung 6c ist wie die Anpassungsstellvorrichtung 6b teleskopstangenartig ausgebildet. Durch die Anpassungsvorrichtung 6c lässt sich die Weite der Haltevorrichtung 1 an eine Dicke des Unterarms 2 anpassen.

Fig. 3 zeigt die Haltevorrichtung aus Fig. 1b mit einem sich in der Haltevorrichtung befindlichen Unterarm 2 in einer vorbestimmten Korrekturstellung in einer Unteransicht. Im Haltebereich 3a befindet sich eine weitere Anpassungsstellvorrichtung 6a mit welcher sich der Haltebereich 3a an eine Größe der Hand anpassen lässt. Die Anpassungsstellvorrichtung 6a ist genauso wie die Anpassungsstellvorrichtungen 6b und 6c teleskopstangenartig ausgebildet, so dass eine Vergrößerung oder Verkleinerung der Länge der Anpassungsstellvorrichtung 6a zu einer Vergrößerung oder Verkleinerung des Abstandes zwischen den Halteelementen 4a führt.

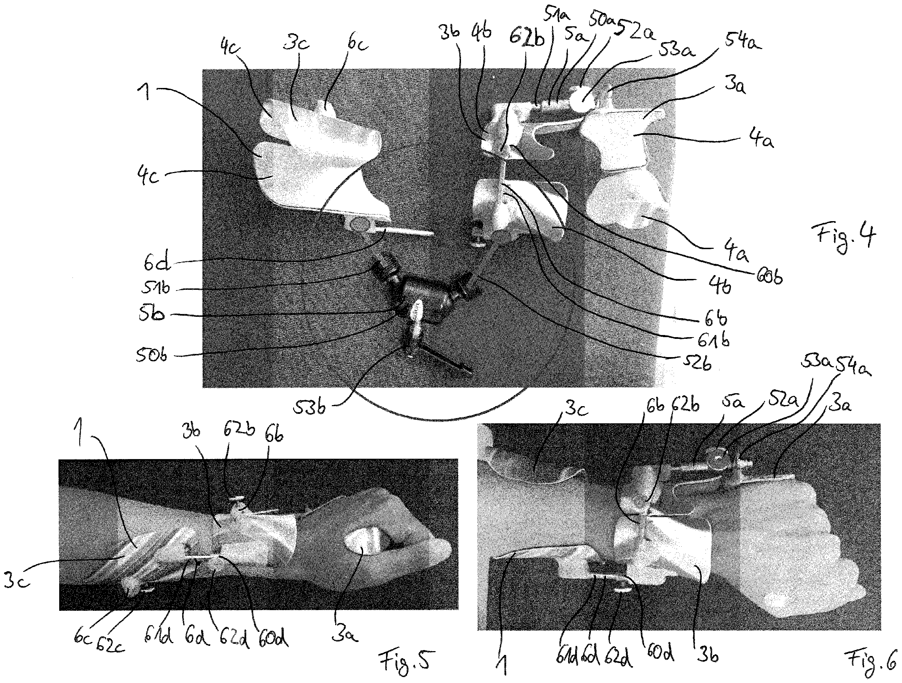

Fig. 4, 5 und 6 zeigen ein weiteres Ausführungsbeispiel einer Haltevorrichtung zum Halten eines Unterarms in einer vorbestimmten Korrekturstellung.

Fig. 4 zeigt eine ähnliche Haltevorrichtung wie in den Fig. 1a, b, 2 und 3 mit drei Haltebereichen 3a, 3b und 3c, die jeweils zwei Halteelemente 4a, 4b und 4c aufweisen. Die Halteelemente 4a, 4b und 4c sind wie in den Fig. 1a, b bis 3 jeweils mit gleichartig ausgebildeten Anpassungsstellvorrichtungen 6a, 6b, 6c verbunden. Wie beispielsweise bei der Anpassungsstellvorrichtung 6b ersichtlich, welche die Halteelemente 4b zum Halten des Unterarms 2 im Bereich des Handgelenks miteinander verbindet, weist die Anpassungsstellvorrichtung 6b ein zylinderförmiges Gehäuse 60b auf, das mit dem auf der Außenseite des Unterarms 2 gelegenen Halteelement 4b fest verbunden ist und in dem eine Stange 61b verschiebbar gelagert ist. Die Stange 61b ist mit dem auf der Innenseite des Handgelenks gelegenen Halteelement 4b fest verbunden. Durch ein Stellrad 62b kann die Einschubtiefe der Stange 61b in das zylinderförmige Gehäuse 60b fixiert werden.

Desweiteren weist die Haltevorrichtung 1 eine Korrekturkraftstellvorrichtung 5a auf, welche die Haltebereiche 3a zum Halten der Handfläche und 3b zum Halten des Handgelenks miteinander verbindet. Ein Teil der Korrekturkraftstellvorrichtung 5a ist ähnlich wie die Anpassungsstellvorrichtungen 6a bis 6c aufgebaut. Die Korrekturkraftstellvorrichtung 5a weist ein zylinderförmiges Gehäuse 50a auf, in dem eine Stange 51a verschiebbar gelagert ist. Die Stange 51a ist fest mit dem außen gelegenen Halteelement 4b verbunden. Eine Einschubtiefe der Stange 51a in das zylinderförmige Gehäuse 50a kann durch ein Stellrad 52a, welches an dem zylinderförmigen Gehäuse 50a außenliegend angebracht ist, fixiert werden. Durch die Änderung der Einschubtiefe der Stange 51a in das zylinderförmige Gehäuse 50a lässt sich ein Abstand zwischen den Haltebereichen 3b und 3a variieren. Das zylinderförmige Gehäuse 50a ist mit einer senkrecht zur Zylinderachse verlaufenden Führungsschiene 53a an seinem dem Haltebereich 3a zugewandten Ende in einer Richtung parallel zur Führungsschiene 54a verschiebbar gelagert. Eine Position des dem Haltebereich 3a zugewandten Endes des zylinderförmigen Gehäuses 50a lässt sich mittels eines Stellrads 53a feststellen. Das Stellrad 53a ist auf einer Oberseite des zylinderförmigen Gehäuses angeordnet. Auf einer Innenseite der Haltevorrichtung 1 befindet sich eine weitere Anpassungsstellvorrichtung 6d, welche die Haltebereiche 3c und 3b miteinander verbindet.

Die Anpassungsstellvorrichtung 6d ist genauso ausgebildet, wie die Anpassungsstellvorrichtung 6a bis 6c und ermöglicht eine Variation des Abstands der Haltebereiche 3c und 3b zueinander und somit eine Anpassung der Haltevorrichtung 1 an eine Größe des in eine Korrekturstellung zu bringenden Unterarms 2. Alternativ kann diese Anpassungsstellvorrichtung 6d durch eine Korrekturkraftstellvorrichtung 5b ersetzt werden. Die Korrekturkraftstellvorrichtung 5b ist analog zu der Korrekturkraftstellvorrichtung 5b der Fig. 1a, b bis 3 ausgebildet. Zusätzlich weist die Korrekturkraftstellvorrichtung 5b einen Hebel 53b zum Fixieren der Stellungen der kugelgelenkartigen Verbindung zwischen den Justierelementen 51b und 52b und dem zylinderförmigen Gehäuse 50b auf.

Auch bei dem Ausführungsbeispiel der Fig. 4 bis 6 können optional Drucksensoren an der Haltevorrichtung befestigt sein. Die konkrete Position der Drucksensoren (in Fig. 4 bis 6 nicht dargestellt) ist abhängig von der Fehlstellung des Unterarms. Die Drucksensoren sind in Bereichen mit großer Hebelwirkung, also maximal entfernt von Umlenkpunkten, und an den Umlenkpunkten selbst angeordnet. Konkret können im Fall von zwei Korrekturkraftstellvorrichtungen 5a, 5b in Fig. 4 drei Drucksensoren wie im Ausführungsbeispiel der Fig. 1b bis 3 an der Haltevorrichtung angeordnet sein. Im Fall von lediglich einer Korrekturkraftstellvorrichtung 5a kann lediglich ein Drucksensor am dem Körper zugewandten Halteelement 4b und ein Drucksensor am dem Körper abgewandten Halteelement 4a angeordnet sein. Fig. 5 zeigt die Haltevorrichtung 1 aus Fig. 4 in einer Innenansicht, wobei die Haltevorrichtung an einem Unterarm angebracht ist.

Fig. 6 zeigt die Haltevorrichtung 1 aus Fig. 4 in einer Draufsicht, wobei die Haltevorrichtung an einem Unterarm angebracht ist.

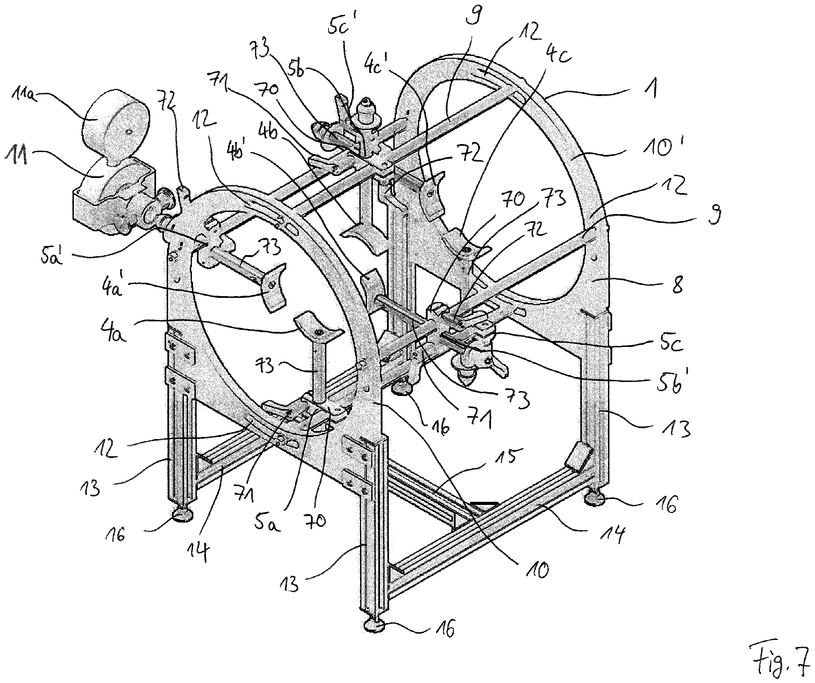

Fig. 7 zeigt ein drittes Ausführungsbeispiel einer Haltevorrichtung in einer Perspektivansicht. Die Haltevorrichtung 1 weist ein Grundgestell 8 auf, welches vier parallel verlaufende Schienen 9 umfasst, die senkrecht zu ihrer Längsrichtung in gleichem Abstand entlang eines Kreises angeordnet sind und einen Aufnahmebereich für einen Unterarm definieren. Mit anderen Worten definieren die Schienen 9 einen Aufnahmebereich für einen Unterarm in einer zu korrigierenden Richtung. Die Schienen 9 sind an ihren Enden mit jeweils einem kreisförmigen Verbindungselement 10, 10' miteinander verbunden. Die Verbindungselemente 10, 10' sind flächig ausgestaltet und weisen kreisflächenförmige Durchgangsöffnungen auf, durch welche ein in eine Korrekturstellung zu bringender Unterarm in den Aufnahmebereich gelangen kann.

In einer oberen Hälfte der Verbindungselemente 10, 10' sind die Verbindungselemente 10, 10' auch an ihrer Außenseite kreisförmig ausgestaltet. In einer unteren Hälfte der Verbindungselemente 10, 10' sind die Verbindungselemente 10, 10' an ihrer Außenseite rechteckig ausgestaltet, so dass eine Unterkante der flächigen Verbindungselemente 10, 10' parallel zu einer Auflagefläche ist, auf der die Haltevorrichtung 1 aufgestellt werden kann. Die Haltevorrichtung 1 verfügt weiter über eine Standvorrichtung, welche vier senkrecht zu den Schienen 9 verlaufende Standschienen 13 umfasst, wobei jeweils zwei Standschienen 13 in der unteren Hälfte eines Verbindungselements 10, 10' an jeweils gegenüberliegenden Seiten angeordnet sind.

Die Verbindungselemente 10, 10' sind in die Schienen 13 von oben hineingeschoben. Die Standschienen 13 sind somit entlang eines rechteckigen Umrisses angeordnet. Entlang der Längsseiten dieses Rechtecks sind die Standschienen 13 mit Stützschienen 14, die parallel zu den Schienen 9 und im Bereich der der Haltevorrichtung 1 abgewandten Enden der Standschienen 13 verlaufen, verbunden. Die Stützschienen 14 sind wiederum durch eine Stützschiene 15, die senkrecht zu den Schienen 9 und zu den Standschienen 13 verläuft, in ihrer Mitte miteinander verbunden. Die Stützschienen 14 und 15 verbessern die Stabilität und die Standfestigkeit der Standvorrichtung der Haltevorrichtung 1. Zusätzlich sind die Standschienen 13 an ihren der Haltevorrichtung 1 abgewandten Enden mit Standfüßen 16 ausgestattet. In den Verbindungselementen 10, 10' sind im gleichen Abstand jeweils vier kreisbogenförmige Aussparungen 12 angeordnet, in welchen die Enden der Schienen 9 zur Verstellung eines Abstands der Schiene 9 zueinander, geführt werden. An den Schienen 9 sind darüber hinaus Korrekturkraftstellvorrichtungen 5a, 5a', 5b, 5b' und 5c, 5c' angeordnet.

Diese Korrekturkraftstellvorrichtungen sind an jeweils zwei zueinander benachbarten Schienen 9 befestigt, so dass jeweils zwei Korrekturkraftstellvorrichtung Korrekturkräfte auf einen Haltebereich ausüben können, wobei die Korrekturkräfte in zwei senkrecht zueinander verlaufende Richtungen ausübbar sind. Die Korrekturkraftstellvorrichtung 5a ist benachbart zum Verbindungselement 10 an der untersten Schiene 9 mittels eines Klemmblocks 70 befestigt. Die Korrekturkraftstellvorrichtung 5a weist eine senkrecht zur Schiene 9 und parallel zu den Standschienen 13 verlaufende Stange 73 auf, die ebenfalls mittels des Klemmblocks 70 befestigt ist. An ihrem dem Aufnahmebereich zugewandten Ende weist die Stange 73 ein Halteelement 4a auf, welches flächig und in Richtung des Aufnahmebereichs konkav zur Auflage eines Unterarms ausgebildet ist.

Ebenfalls benachbart zum gleichen Verbindungselement 10 und auf Höhe der Korrekturkraftstellvorrichtung 5a ist eine weitere Korrekturkraftstellvorrichtung 5a' an einer der untersten Schiene 9 benachbarten Schiene 9 befestigt mittels eines Klemmblocks 70. Die Korrekturkraftstellvorrichtung 5a' weist ebenfalls eine Stange 73 auf, die jedoch nun senkrecht zur Schiene 9 und parallel zur Stützschiene 15 ebenfalls mittels des Klemmblocks 70 befestigt ist. An ihrem dem Aufnahmebereich zugewandten Ende weist die Stange 73 ein in Richtung des Aufnahmebereichs konkav gewölbtes, flächig ausgebildetes Halteelement 4a' zum Halten eines Unterarms auf. Die beiden Halteelemente 4a und 4a' bilden so einen Haltebereich 3a (hier nicht eingezeichnet).

Mittels der Drehhebel 71 lässt sich die Länge der Stange 73, mit welcher die Stange 73 in den Aufnahmebereich hineinragt, verändern und fixieren. Mithilfe eines Drehhebels 72 lässt sich die Position der Korrekturkraftstellvorrichtung 5a, 5a' entlang der Schiene 9 verändern und feststellen. Durch Aufdrehen der Hebel 71 oder 72 verringert sich die Klemmung des Klemmblocks 70, wodurch sich der Klemmblock 70 entlang der Schiene 9 verschieben lässt bzw. sich die Stange 73 senkrecht zur Schiene 9 verschieben lässt.

An ihrem dem Aufnahmebereich abgewandten Ende ist ein Kraftmesssensor 11 an die Korrekturkraftstellvorrichtung 5a, 5a' zur Messung der von der Korrekturkraftstellvorrichtung auf einen Unterarm in einer Richtung parallel zur Stützschiene 15 ausgeübten Korrekturkraft angebracht. Ferner verfügt der Kraftmesssensor 11 über eine Anzeige 11a, auf welcher die ausgeübte Korrekturkraft angezeigt werden kann. In etwa mittig zwischen den Verbindungselementen 10, 10' ist auf einer obersten Schiene 9 eine weitere Korrekturkraftstellvorrichtung 5b angeordnet, welche eine Korrekturkraft nach unten und parallel zu den Standschienen 13 ausüben kann.

An einer zu dieser Schiene 9 benachbarten Schiene 9 ist eine weitere Korrekturkraftstellvorrichtung 5b' angeordnet, welche ausgebildet ist, eine Korrekturkraft parallel zur Stützschiene 15 auszuüben. Benachbart zum Verbindungselement 10' ist eine weitere Korrekturkraftstellvorrichtung 5c an der untersten Schiene 9 angeordnet, welche ausgebildet ist eine Korrekturkraft parallel zu den Stützschienen 13 nach oben auszuüben. An einer zu dieser Schiene 9 benachbarten Schiene 9 ist eine weitere Korrekturkraftvorrichtung 5c' angeordnet, welche ausgebildet ist eine Korrekturkraft parallel zur Stützschiene 15, in die gleiche Richtung wie die Korrekturkraftstellvorrichtung 5a', auszuüben. Sämtliche Korrekturkraftstellvorrichtungen 5a, 5a', 5b, 5b', 5c, 5c' weisen die gleichen Bestandteile auf. Sie unterscheiden sich lediglich in ihrer Wirkrichtung und in der Schiene 9, an welcher sie angebracht sind.

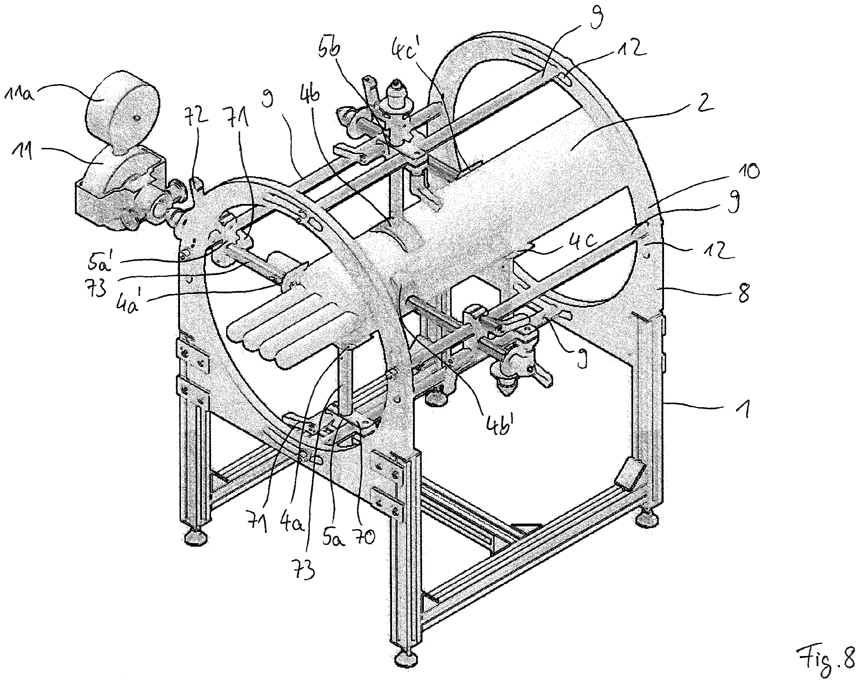

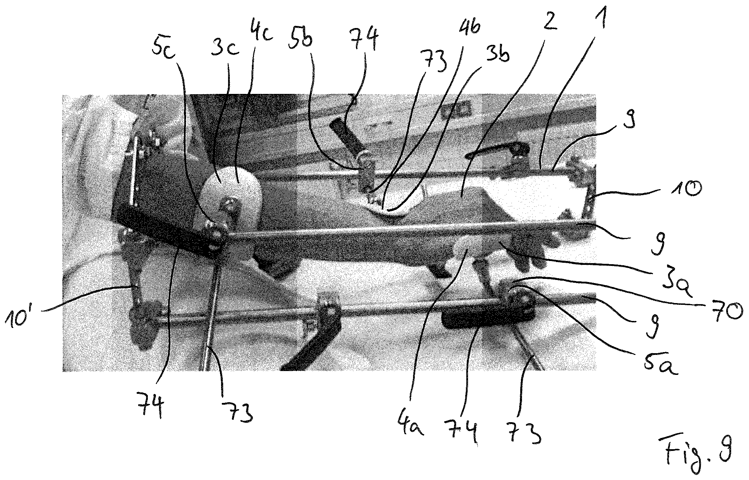

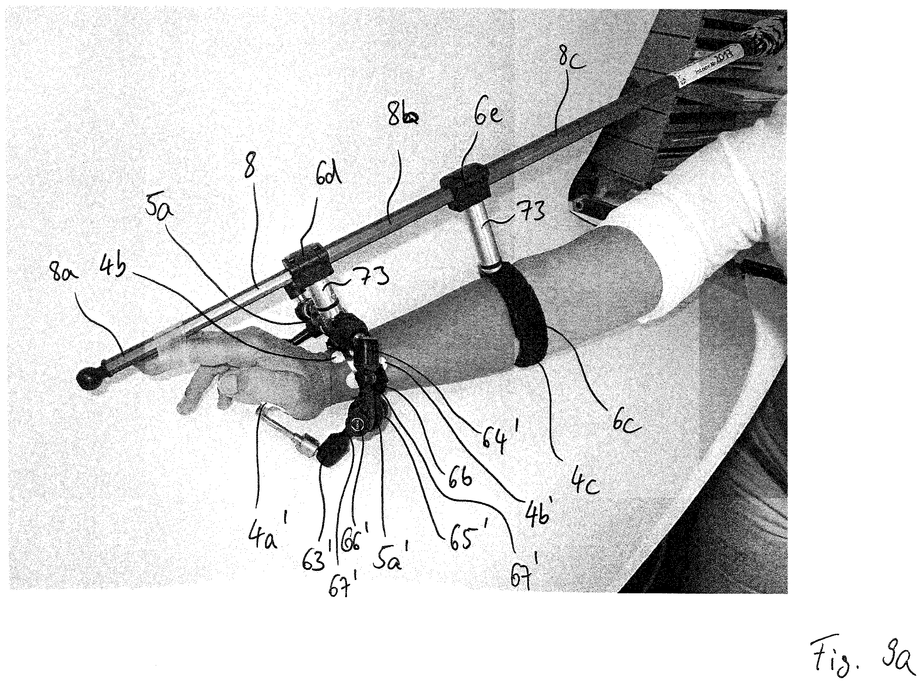

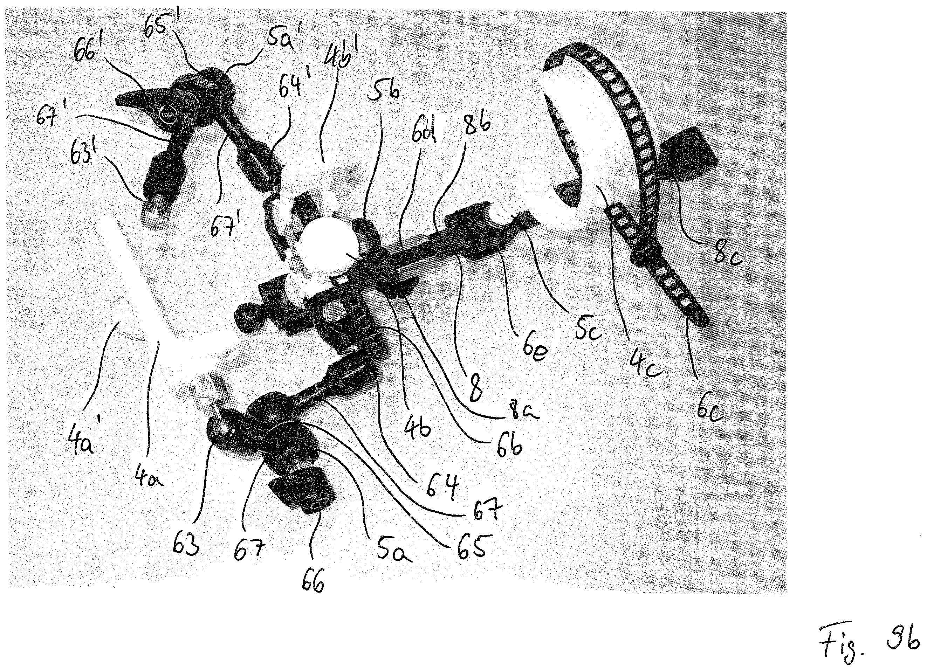

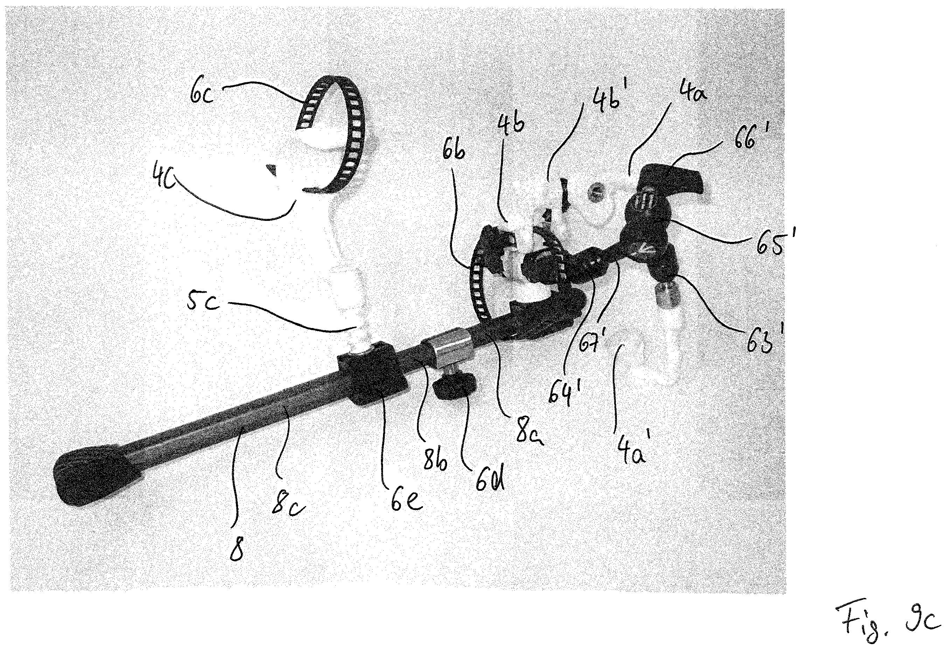

Fig. 8 zeigt die Haltevorrichtung 1 aus Fig. 9, wobei nun ein Unterarmmodell 2 von der Haltevorrichtung 1 gehalten wird. Die Halteelemente 4a' und 4a stützen dabei den Handbereich des Unterarmmodells 2, die Halteelemente 4b, 4b' halten das Unterarmmodell 2 im Handgelenk-nahen Bereich des Unterarms und die Halteelemente 4c, 4c' halten das Unterarmmodell 2 im Bereich des Unterarmknochens. Mittels der Haltevorrichtung 1 ist somit eine Korrektur der Stellung des Unterarms in allen drei Raumrichtungen möglich.

Fig. 9 zeigt ein weiteres Ausführungsbeispiel einer Haltevorrichtung zum Halten eines Unterarms in einer vorbestimmten Korrekturstellung. Die Haltevorrichtung 1 weist vier parallel zueinander verlaufende Schienen 9 auf, welche durch Verbindungselemente 10, 10' entlang eines Umfangs im Bereich ihrer Enden miteinander verbunden sind. Die Schienen 9 und die Verbindungselemente 10, 10' bilden ein Grundgestell der Haltevorrichtung 1. Die Verbindungselemente 10 und 10' sind jeweils aus Lochschienen zusammengesetzt, wobei zwei in senkrechter Richtung benachbarte Schienen 9 durch eine einteilige Lochschiene verbunden sind und zwei in horizontaler Richtung benachbarte Schienen durch drei aneinander geschraubte Lochschienen verbunden sind. Diese drei Lochschienen können variabel miteinander verschraubt werden, so dass die Verbindung der horizontal benachbarten Schienen 9 in der Länge variabel ist.

An den Schienen 9 sind Korrekturkraftstellvorrichtungen 5a bis 5c angeordnet. Benachbart zu dem ersten Verbindungselement c ist eine Korrekturkraftstellvorrichtung 5a angeordnet, welche an ihrem einem Aufnahmebereich für den Unterarm 2 zugewandten Ende einen Haltebereich 3a aufweist. Dieser Haltebereich 3a hält die Handfläche des Unterarms 2. Das den Haltebereich 3a bildende Halteelement 4a ist mit einer Stange 73 verbunden, welche senkrecht zu den Schienen 9 verläuft und mittels eines Klemmblocks 70 an der Schiene 9 befestigt ist. Durch Lösen und Verschieben des Klemmblocks 70 lässt sich die Korrekturkraftstellvorrichtung 5a entlang der Schiene 9 verschieben. Zum Feststellen des Klemmblocks 70 ist ein Schnellspanner 74 am Klemmblock 70 angeordnet.

Desweiteren befindet sich eine gleichartig ausgebildete Korrekturkraftstellvorrichtung 5b im Handgelenk-nahen Bereich des Unterarms auf einer zur Schiene 9, an welcher die Korrekturkraftstellvorrichtung 5a angeordnet ist, diagonal gegenüberliegenden Schiene 9 des Grundgestells der Haltevorrichtung 1. Desweiteren befindet sich eine gleichartig ausgebildete Korrekturkraftstellvorrichtung 5c im Bereich des Unterarmknochens auf einer Schiene 9, welche sich auf der gleichen Seite wie die Korrekturkraftstellvorrichtung 5a befindet, jedoch auf einer anderen Schiene befestigt ist als die Korrekturkraftstellvorrichtung 5a.

Fig. 9a zeigt ein weiteres Ausführungsbeispiel einer Haltevorrichtung, in der ein rechter Unterarm gehalten wird. Die Haltevorrichtung weist als Grundgestell eine einzige Teleskopstange 8 auf, an welcher die Halteelemente 4a, 4a', 4b und 4c der Reihe nach befestigt sind. Die Teleskopstange 8 weist drei ineinander verschiebbare Elemente 8a, 8b, 8c auf. Das Halteelement 4c, welches den Unterarm im Bereich des Ellenbogens hält, ist über eine zylinderförmige Stützstange 73 und einen Schnellverschluss 6e mit dem äußersten Element 8c der Teleskopstange 8 verbunden. Das Halteelement 4c ist dabei in Form eines Halbrings ausgebildet und geeignet den Unterarm von einer Seite sowie einer Ober- und einer Unterseite zu umgreifen. Mit dem Schnellverschluss 6e lässt sich eine Position des mittleren Elements 8b relativ zum äußersten Element 8c und damit der Abstand zwischen den Haltebereichen zum Halten des Unterarms im Bereich des Handgelenks und zum Halten des Unterarms im Bereich des Ellenbogens fixieren. Die Halteelemente 4a, 4a', 4b, 4b' sind über eine zylinderförmige Stützstange 73 und einen Schnellverschluss 6d mit dem mittleren Element 8b der Teleskopstange 8 verbunden. Die Halteelemente 4b, 4b' stützen den Unterarm im Bereich des Handgelenks von einer Oberseite des Unterarms sowie von einer Seite ab, welche der Seite gegenüberliegt, von der aus das Halteelement 4c den Unterarm umgreift. Mit dem Schnellverschluss 6d lässt sich eine Position des innersten Elements 8a der Teleskopstange 8 relativ zum mittleren Element 8b fixieren. Am innersten Element 8a besteht die Möglichkeit den Unterarm zusätzlich im Bereich der Finger zu befestigen. Die Halteelemente 4b, 4b' und 4c weisen jeweils Klettbänder 6b und 6c zur Befestigung des Unterarms auf. Zur Befestigung des Unterarms werden die Klettbänder um den Unterarm jeweils im Bereich des Handgelenks und im Bereich des Ellbogens gelegt. Die Halteelemente 4b, 4b' zum Halten des Unterarms im Bereich des Handgelenks sind starr mit der Stützstange 73 und dem Schnellverschluss 6d verbunden. Die Halteelemente 4b, 4b' stützen den Unterarm im Bereich des Handgelenks von einer Oberseite des Unterarms sowie von einer Seite ab, welche der Seite gegenüberliegt, von der aus das Halteelement 4c den Unterarm umgreift. Die Halteelemente 4a, 4a' sind über mehrgelenkige Arme, welche Korrekturkraftvorrichtungen 5a und 5a' bilden, mit der Stützstange 73 und dem Schnellverschluss 6d ausrichtbar verbunden. Diese Arme weisen von der Stützstange 73 wegführend jeweils ein Kugelgelenk 64, 64', einen Gelenkarm 67, 67', ein Scharniergelenk 65, 65', einen weiteren Gelenkarm 67, 67' und ein weiteres Kugelgelenk 63, 63' auf. Am Scharniergelenk 65, 65' ist ein Schnellverschlusshebel 66, 66' angeordnet, welcher ein gleichzeitiges Arretieren aller Gelenke des Armes ermöglicht. Hierdurch kann eine gewünschte Position der Korrekturkraftvorrichtungen 5a, 5a' schnell und einfach fixiert werden. Halteelemente 4a und 4a' stützen die Handseiten- sowie -innenfläche und Daumen unabhängig voneinander ab, sodass mittels der mehrgelenkigen Arme 5a, 5a' eine gewünschte Stellung des Handgelenks einstellbar ist.

Fig. 9b zeigt eine Weiterentwicklung der Haltevorrichtung aus Fig. 9a als ein weiteres Ausführungsbeispiel. Im Unterschied zur Haltevorrichtung aus Fig. 9a weist die Haltevorrichtung im dritten Haltebereich (im Bereich des Ellenbogens) anstatt der Stützstange 73 ein Gewinde als eine weitere Korrekturkraftstellvorrichtung 5c auf. Das Gewinde ermöglicht es, den Abstand des Halteelements 4c von der Teleskopstange 8 zu vergrößern oder zu verkleinern und damit einen Winkel zwischen dem Unterarm und der Teleskopstange 8, also den Winkel, in welchem der Unterarm in der Haltevorrichtung gehalten wird, zu variieren. Dies ermöglicht die Stellung des Handgelenks noch stärker variieren zu können. Ferner weisen die Halteelemente 4c und 4b, 4b' elastische Löchbänder 6b und 6c als Anpassungsstellvorrichtungen auf.

Fig. 9c zeigt die Haltevorrichtung aus Fig. 9b in einer weiteren Perspektivansicht.

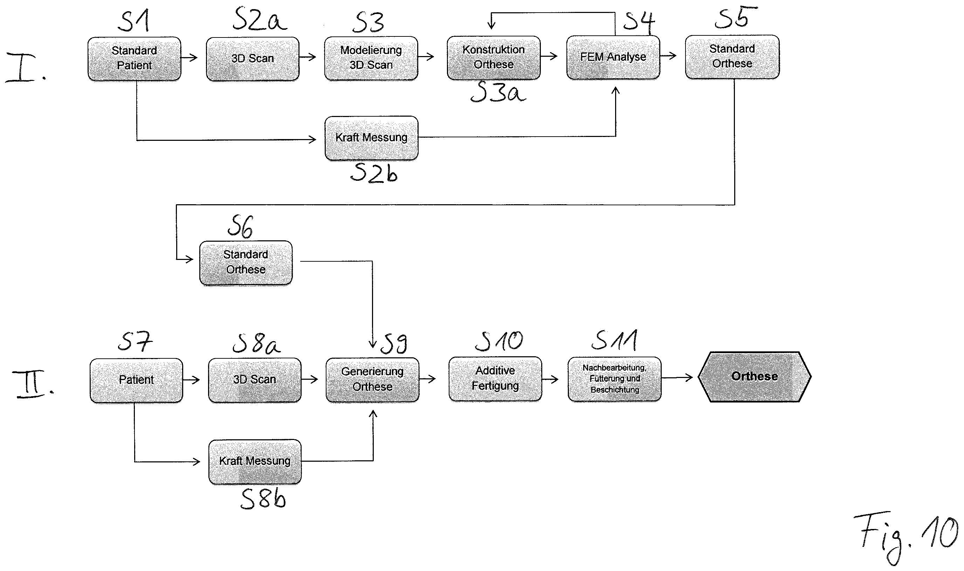

Als nächstes wird ein erfindungsgemäßes Verfahren zur Herstellung einer Orthese anhand von Fig. 10 detailliert beschrieben. Konkret wird die Herstellung einer Unterarmspiralorthese beschrieben, wobei der Herstellungsprozess in zwei Teilprozesse aufgespalten werden kann. Als erstes wird der Entwicklungsprozess zur Entwicklung eines Standardorthesenmodells beschrieben (Teilprozess I in Fig. 10).

Zunächst wird mittels einer Haltevorrichtung der Unterarm in der gewünschten Korrekturstellung fixiert. Hierzu werden die sich an der Haltevorrichtung befindlichen Halteelemente an den Stellen des Unterarms positioniert, an denen auch die späteren Kraftaufnahmeflächen der hergestellten Unterarmspiralprothese positioniert sein werden. Dadurch wird der Unterarm in der gewünschten Funktionalstellung (Korrekturstellung) platziert (S1). Anschließend wird mithilfe eines 3D-Scanners der komplette Unterarm des Patienten digitalisiert (S2a). Zusätzlich wird unter Verwendung von Druckmesssensoren, die auf den Körperteilkontaktflächen der Halteelemente befestigt oder in die Halteelemente integriert sind, die vom Patienten erzeugbare und auf die spätere Orthese ausübbare Kraft ermittelt (S2b). Als nächstes wird die entsprechende Standardorthese als Formgebungsmodell sowie die Struktur modelliert (S3) und auf den 3D-Scan des Unterarms des Patienten konstruiert (S3a). Mithilfe der zuvor ermittelten vom Unterarm auf die Halteelemente ausgeübten Kräfte wird eine FEM-Analyse durchgeführt (S4). Hiermit kann die bisherige Geometrie der Spiralorthese belastungstechnisch validiert werden. Sollte unter Berücksichtigung der begrenzenden Designkriterien noch Optimierungspotential bei der Geometrie der Orthese zur Erhöhung der Stabilität bestehen, kann an dieser Stelle durch ein erneutes, verbessertes Design die Form des Orthesenmodells angepasst werden (S3a). Die Modellierung einer Gitterstruktur erfolgt dabei nach Maßgabe einer optimalen Belüftung der Haut sowie einer Gewichtsoptimierung. Dieses Design wird nun als Standarddesign verwendet (S5), die Grundgeometrie wird im Folgenden nicht mehr verändert.

An dieser Stelle der Entwicklung ist ein Orthesenmodell vorhanden, dasan einen 3D-Scan eines Unterarms angepasst werden kann, sowie mithilfe von belastungstechnischen Parametern deren Größe sowie die Struktur sich variabel einstellen lässt (S6).

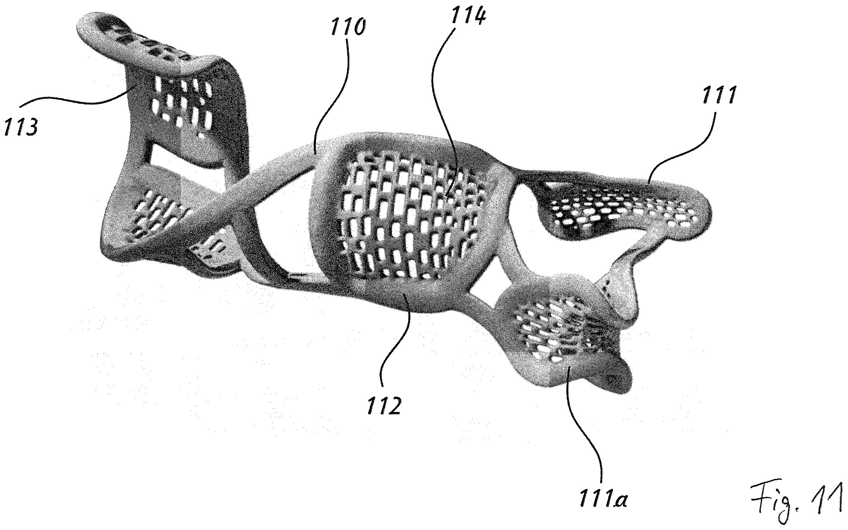

Der Teilprozess zur Herstellung einer Spiralorthese (Teilprozess II in Fig. 10) stellt sich nun in folgender Weise dar: Zunächst wird der Unterarm des Patienten mittels der Haltevorrichtung in die Korrekturstellung gebracht (S7). Mithilfe des 3D-Scanners wird eine digitale Kopie des Unterarms erstellt (S8a). Zusätzlich wird durch die an der Haltevorrichtung befindlichen Druckmesssensoren die vom Patientenunterarm auf die spätere Orthese ausübbare Kraft erfasst (S8b). Unter Verwendung des StandardOrthesenmodells für die Spiralorthese kann ein patientenindividuelles Orthesenmodell generiert werden (S9). Auf Basis dieses StandardOrthesenmodells kann die Orthese mittels eines additiven Fertigungsverfahrens ausgedruckt werden (S10). Nach dem Ausdrucken wird die Oberfläche der Orthese geglättet, beispielsweise durch Trovalisierung, und schließlich kann noch eine Polsterung und/oder Fütterung aufgebracht werden (S11). Zusätzlich kann eine Oberflächenbehandlung wie Lackieren, Beschichten, Beziehen mit Stoff, Leder oder sonstigen Materialien durchgeführt werden. Fig. 11 zeigt ein fertiges drei-dimensionales Modell einer Unterarmspiralorthese (UASO) 110 zur Korrektur einer Fehlstellung eines Unterarms, wie es nach Beendigung des Prozessschritts S6 bzw. S9 der Figur 10 vorliegt.
Das Orthesenmodell 110 weist drei Stützbereiche 111 bis 113 auf, wobei jeder Stützbereich derart gestaltet ist, dass er auf einen Unterarm Korrekturkräfte in mindestens zwei verschiedenen Bewegungsebenen des Handgelenks ausüben kann.

Ein erster Stützbereich 111 ist entlang der Handinnenfläche eines zu stützenden Unterarms ausgebildet. Dieser Stützbereich erstreckt sich weiter entlang einer Außenseite des kleinen Fingers bis zum Handgelenk. Ferner erstreckt sich der Stützbereich 111 zwischen dem Zeigefinger und Daumen zum Handgelenk. Zusätzlich ist der Stützbereich 111 auf der Handinnenfläche im Bereich des Daumengrundgelenks zu einer Daumenführung 111a zur Korrektur und Stützung der Daumenstellung ausgeformt. Ein zweiter Stützbereich 112 erstreckt sich an einer der Handinnenfläche abgewandten Oberseite des Handgelenks bis zu den Seitenbereichen des Handgelenks. Der Bereich der Unterseite des Handgelenks ist dabei ausgespart. Entlang einer dem Körper zugewandten Innenseite schließt sich an den zweiten Stützbereich 112 ein dritter Stützbereich 113 an. Der Stützbereich 113 erstreckt sich weiter entlang einer der Oberseite des Handgelenks abgewandten Unterseite des Unterarms bis zu einer vom Körper abgewandten Außenseite des Unterarms.

Das UASO-Modell 110 weist somit gemäß dem Drei-Punkte-Prinzip jeweils drei Stützbereiche 111, 112 und 113 bezüglich zweier Bewegungsebenen des Handgelenks auf. Das Drei-Punkte-Prinzip besagt, dass drei Stützbereiche notwendig sind, um eine Korrektur der Fehlstellung in einer Bewegungsebene zu erreichen.

Eine einen Unterarm haltende Spiralorthese mit der vorstehend beschriebenen Orthesengeometrie ermöglicht somit die Ausübung der folgenden Korrekturkräfte:
Bezüglich einer Bewegung des Handgelenks senkrecht zur Ebene der Handfläche ist der erste Stützbereich 111 ausgebildet, im Bereich der Finger- und Daumengrundgelenke im Wesentlichen eine Korrekturkraft gegen die Handinnenfläche auszuüben. Des Weiteren ist der Stützbereich 112 ausgebildet, auf der Oberseite des Handgelenks eine im Wesentlichen senkrecht auf die Oberseite des Handgelenks gerichtete Korrekturkraft auszuüben. Der dritte Stützbereich 113 ist ausgebildet, auf der Unterseite des Unterarms auf der zum Körper hin gelegenen (proximalen) Seite der Orthese 110 eine Korrekturkraft senkrecht auf die Unterseite des Unterarms auszuüben.

Bezüglich einer Bewegung des Handgelenks in einer Ebene der Handfläche ist der Stützbereich 111, welcher sich bei angelegter UASO 110 auch entlang der Außenseite des kleinen Fingers erstreckt, eine Korrekturkraft senkrecht auf die Außenseite des kleinen Fingers auszuüben. Des Weiteren ist der Stützbereich 112, welcher sich bei angelegter UASO 110 auch über die zur Körpermitte hin gelegene Innenseite des Handgelenks erstreckt, eine Korrekturkraft senkrecht auf die Innenseite des Handgelenks auszuüben. Außerdem ist der Stützbereich 113, welcher sich bei angelegter UASO 110 auch entlang einer körperabgewandten Außenseite des Unterarms erstreckt, eine Korrekturkraft senkrecht auf die Außenseite des Unterarms auszuüben.

Die UASO weist zusätzlich eine Daumenführung 111a auf, welche bei angelegter UASO zwischen Zeigefinger und Daumen vom Handrücken zur Handinnenfläche verläuft und welche die Lage des Daumens stützen und korrigieren soll.
Die Geometrie der Orthese wurde mittels einer FEM-Simulation (Prozessschritt S4 in Fig. 10) belastungstechnisch sowie gewichtstechnisch optimiert, wie im Folgenden kurz beschrieben wird. Anhand einer FEM-Simulation kann die Belastung entlang der Orthesengeometrie analysiert und dadurch mögliche über- und unterdimensionierte Bereiche der Orthesengeometrie ermittelt werden. Hierzu wird zunächst das Lastbild definiert. Benötigt werden die Höhe der wirkenden Kraft, die Wirkorte und die Größe der betroffenen Flächen sowie mindestens eine Stelle, an der die Orthese fixiert ist. Unter realistischen Gesichtspunkten müsste die gesamte an der Orthese anliegende Fläche des Unterarms bei dem Beugevorgang betrachtet werden, da sich die wirkenden Kräfte auf die gesamten Kontaktbereiche zwischen Haut und Orthese aufteilen. Um die Komplexität der Simulation möglichst gering zu halten, werden vereinfacht die auf die Orthese wirkenden Kräfte nur in den Stützbereichen des Drei-Punkte-Prinzips angenommen. Zur Bestimmung der von den Patienten auf die spätere Orthese ausübbaren Kräfte werden die an den Halteelementen befestigten Kraftmesssensoren bzw. die in die Halteelemente integrierten Kraftmesssensoren verwendet. Nach der durchgeführten Messung werden die vom Patienten maximal erzeugbaren Kräfte anhand des Messresultats ausgelesen. Zusätzlich können als grobe Richtwerte die durch Erfahrung festgelegten Werte der Orthopädietechnik dienen. Hierbei beträgt der maximal als nicht unangenehm empfundene Druck in Bereichen mit weichem, dick ausgeprägtem Gewebe 40 N/mm², in Bereichen mit dünnem Gewebe und der darunter befindlichen harten Knochen 25 N/mm². Anhand der durchgeführten Simulation wird insbesondere im Bereich der Verbindung zwischen vorderem und hinterem Teilbereich der Orthese, also zweitem Stützbreich 112 und drittem Stützbereich 113, eine erhöhte Belastung des Materials deutlich. Ferner wird ebenfalls eine erhöhte Belastung im ersten Stützbereich 111 im Bereich der Handinnenfläche festgestellt. Nur sehr gering belastet wird hingegen der Ausläufer der Spirallasche am Unterarm, also der dritte Stützbereich 113. Hierbei muss allerdings die funktionale Notwendigkeit berücksichtigt werden, dass ein dritter Stützbereich vorhanden sein muss.

Die Wandung der UASO 110 weist im ersten 111, zweiten 112 und dritten Stützbereich 113 eine Gitterstruktur 114 auf, welche ebenfalls entsprechend der berechneten Belastungsverteilung entlang der Wandung der UASO 110 modelliert ist.

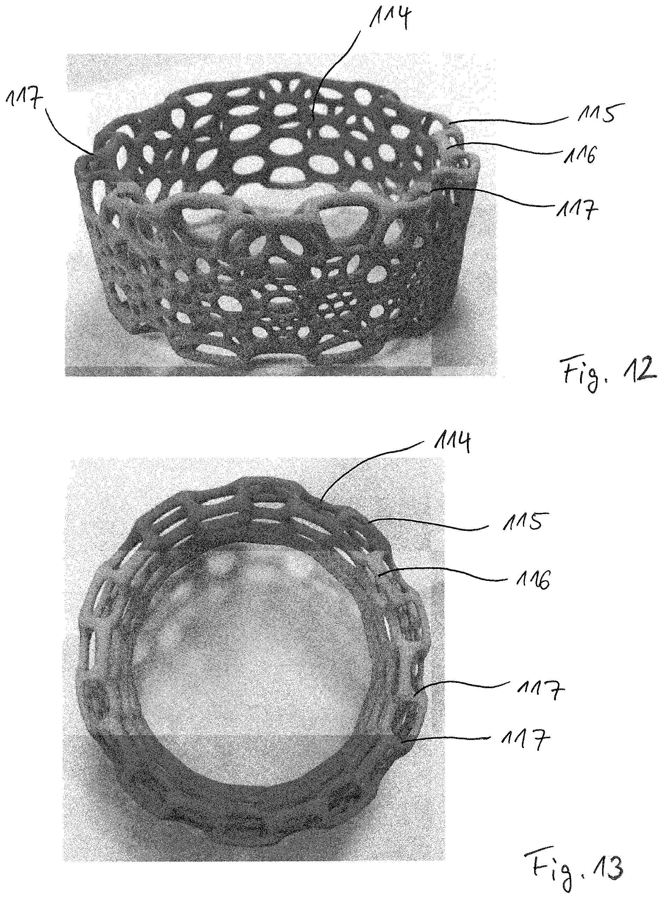

Fig. 12 und 13 zeigen ein weiteres Beispiel für eine mögliche Gitterstruktur 114, die in einer Orthese vorgesehen sein kann, in einer Perspektivansicht (Fig. 12) und einer Draufsicht (Fig. 13). Bei der dargestellten Gitterstruktur 114 handelt es sich um eine dreidimensionale Struktur in Form einer zylindrischen Mantelfläche mit doppelter Wandung. Mit anderen Worten handelt es sich um zwei zwei-dimensionale Gitterebenen, eine äußere Gitterebene 115 und eine innere Gitterebene 116, die sich in etwa entlang einer Zylindermantelfläche erstrecken und in radialer Richtung durch Gitterstege 117 miteinander verbunden sind.

## Patentansprüche

1. Verfahren zur Herstellung einer Orthese zur Korrektur einer Fehlstellung eines Körperteils, wobei die Orthese mittels einer Vorrichtung zur Herstellung der Orthese hergestellt wird,
wobei die Vorrichtung zur Herstellung der Orthese aufweist:
eine Vorrichtung zum Erfassen von Patientendaten und eine Datenverarbeitungseinrichtung zum Erzeugen eines digitalen Orthesenmodells und zum Erzeugen eines angepassten Orthesenmodells der Orthese, in dem das digitale Orthesenmodell basierend auf den Patientendaten an den Körperteil individuell angepasst wird,
und eine Fertigungsvorrichtung zur Fertigung der Orthese basierend auf dem angepassten Orthesenmodell, wobei die Patientendaten Geometriedaten zur Beschreibung einer Geometrie des Körperteils umfassen,
wobei die Vorrichtung zur Erfassung von Patientendaten aufweist:
eine Haltevorrichtung (1) zum Halten des Körperteils in einer einstellbaren Korrekturstellung, ein Scangerät zum Scannen der Geometrie des von der Haltevorrichtung (1) gehaltenen Körperteils und Erzeugen der Geometriedaten und ein Speichergerät zum Speichern der Patientendaten,
wobei die Haltevorrichtung
eine Mehrzahl von Haltebereichen (3a-c) und eine oder mehrere Korrekturkraftstellvorrichtungen (5a-c, 5a'-c') aufweist, wobei mindestens eine der Korrekturkraftstellvorrichtungen (5a-c, 5a'-c') mit mindestens einem der Haltebereiche (1) verbunden ist, und mittels der Korrekturkraftstellvorichtungen (5a-c, 5a'-c') Korrekturkräfte über die mit der einen oder mehreren Korrekturkraftstellvorrichtungen (5a-c, 5a'-c') verbundenen Haltebereichen (3a-c) auf den Körperteil ausübbar sind, und hierdurch der Körperteil in die Korrekturstellung bringbar ist,
und wobei jeder Haltebereich (3a-c) ein, zwei oder mehrere Halteelemente (4a-c, 4a'-c') zum Fixieren des Körperteils in jeweils mindestens einer Raumrichtung und/oder zum Übertragen der mittels der Korrekturkraftstellvorrichtungen (5a-c, 5a'-c') ausgeübten Korrekturkräfte an den Körperteil aufweist,
wobei das Verfahren die folgenden Schritte aufweist:
i) der Körperteil wird in einer eingestellten Korrekturstellung in der Haltevorrichtung gehalten,
ii) die Geometrie des von der Haltevorrichtung (1) gehaltenen Körperteils wird von dem Scangerät gescannt,
iii) die Geometriedaten des in der Haltevorrichtung gehaltenen Körperteils werden erzeugt und als Patientendaten in dem Speichergerät gespeichert,
iv) ein digitales Orthesenmodell der Orthese für den Körperteil erzeugt wird und basierend auf den Patientendaten an den Körperteil individuell angepasst und
v) die Orthese wird basierend auf dem angepassten Orthesenmodell gefertigt.

2. Verfahren nach dem vorhergehenden Anspruch, **dadurch gekennzeichnet, dass** die Haltevorrichtung (1) einen oder mehrere Kraftsensoren (11) zum Messen der auf den Körperteil ausgeübten Korrekturkräfte aufweist und mindestens einer der Kraftsensoren (11) in eines der Halteelemente (4a-c, 4a'-c') oder eine Mehrzahl der Kraftsensoren (11) in einige der oder alle Halteelemente (4a-c, 4a'-c') integriert sind.

3. Verfahren nach dem vorhergehenden Anspruch, **dadurch gekennzeichnet, dass** die Kraftsensoren (1) Drucksensoren oder Druckmessgeräte umfassen.

4. Verfahren nach einem der vorhergehenden Ansprüche, **dadurch gekennzeichnet, dass** mindestens eine der Korrekturkraftstellvorrichtungen (5a-c, 5a'-c') eine längenverstellbare Stange (73), eine Teleskopstange, eine Linearführung, einen Linearzylinder (50a), jeweils zwei Schenkel (51b, 52b), deren Winkelstellung justierbar ist, einen mehrgelenkigen Arm (63-67, 63'-67') und/oder ein justierbares Scherengelenk aufweisen.

5. Verfahren nach dem vorhergehenden Anspruch, **dadurch gekennzeichnet, dass** die mindestens eine Korrekturkraftstellvorrichtung (5a-c, 5a'-c') einen drei-gelenkigen Arm (63-67), 63'.67') aufweist, der ein mittleres und zwei äußere Gelenke aufweist, wobei das mittlere Gelenk ein Scharniergelenk (65, 65') ist und die äußeren Gelenke Kugelgelenke (63,63', 64, 64') sind, und wobei mindestens eines der äußeren Gelenke mit einem der Haltebereiche (3a-c) verbunden ist, und durch Variation einer Stellung der äußeren und des mittleren Gelenks Korrekturkräfte ausübbar sind.

6. Verfahren nach einem der vorhergehenden Ansprüche, **dadurch gekennzeichnet, dass** zwei Haltebereiche (3a-c) über eine Korrekturkraftstellvorrichtung (5a-c, 5a'-c') miteinander verbunden sind, mittels welcher eine Korrekturkraft auf einen ersten Haltebereich (3a-c) relativ zu einem zweiten Haltebereich (3a-c) ausübbar ist.

7. Verfahren nach einem der Ansprüche 1 bis 5, **dadurch gekennzeichnet, dass** die Haltevorrichtung (1) ein Grundgestell (8) aufweist, welches einen Aufnahmebereich für den Körperteil definiert und an dem die Korrekturkraftstellvorrichtungen (5a-c, 5a'-c') befestigt sind, welche sich an dem Grundgestell (8) abstützen und geeignet sind, Korrekturkräfte relativ zum Grundgestell (8) über die mit den Korrekturkraftstellvorrichtungen (5a-c, 5a'-c') verbundenen Haltebereiche (3a-c) auf den Körperteil auszuüben.

8. Verfahren nach dem vorhergehenden Anspruch, **dadurch gekennzeichnet, dass** das Grundgestell (8) eine Schiene, eine Stange oder eine Teleskopstange (8a-c) ist, oder das Grundgestell in parallel oder in einem Winkel zueinander verlaufende Schienen (9) umfasst, welche in einer zu korrigierenden Richtung den Aufnahmebereich definieren und an welchen die Korrekturkraftstellvorrichtungen (5a-c, 5a'-c') befestigt sind, wobei ein gemeinsamer Querschnitt durch die Schienen (9) senkrecht zur Längsausdehnung der Schienen (9) ein regelmäßiges oder unregelmäßiges n-Eck bildet, und wobei zueinander benachbarte Schienen (9) durch mindestens ein Verbindungselement (10, 10') miteinander starr verbunden sind, und wobei n ≥ 3.

9. Verfahren nach einem der zwei vorhergehenden Ansprüche, **dadurch gekennzeichnet, dass** die Korrekturkraftstellvorrichtungen (5a-c, 5a'-c') relativ zum Grundgestell (8) verschiebbar und/oder verdrehbar befestigt sind und Positionen der Korrekturkraftstellvorrichtungen (5a-c, 5a'-c') auf den Schienen (9) einstellbar sind, insbesondere mittels Schnellspannern (74), Stellschrauben, Lochschienen und/oder Rastelementen.

10. Verfahren nach einem der zwei vorhergehenden Ansprüche, **dadurch gekennzeichnet, dass** mindestens eine der Korrekturkraftstellvorrichtungen (5a-c, 5a'-c') eine längenverstellbare Stange (73), insbesondere eine Gewindestange, aufweist und derart an den Schienen (9) befestigt ist, dass eine Längsachse der längenverstellbaren Stange (73) weitgehend senkrecht zu den Schienen (9) ausgerichtet ist und durch eine Variation der Länge der Stange (73) Korrekturkräfte auf den Körperteil variierbar sind.

11. Verfahren nach einem der vorhergehenden Ansprüche, **dadurch gekennzeichnet, dass** die Korrekturkraftstellvorrichtungen (5a-c, 5a'-c') mittels eines Schnellspanners (74) oder eines Schnellverschlusses (66, 66') zur Ausübung einer bestimmten Korrekturkraft arretierbar sind.

12. Verfahren nach einem der Ansprüche 8-10, **dadurch gekennzeichnet, dass** die Schienen (9) und/oder die Halteelemente (4a-c, 4a'-c') formstabile Materialien, insbesondere Metall oder Kunststoff, insbesondere thermoplastische, duroplastische und/oder elastomere Kunststoffe oder Glas aufweisen oder daraus bestehen.

13. Verfahren nach einem der vorhergehenden Ansprüche, **dadurch gekennzeichnet, dass** die Patientendaten Kraftmessdaten zur Beschreibung der von den Korrekturkraftstellvorrichtungen (5a-c, 5a'-c, 5a'-c') auf den Körperteil ausgeübten Korrekturkräfte umfassen.

## Claims

1. A method for producing an orthosis for correcting a malposition of a body part, wherein the orthosis is produced by means of a device for producing the orthosis,
wherein the device for producing the orthosis has:
a device for capturing patient data and a data-processing device for generating a digital orthosis model and for generating an adapted orthosis model of the orthosis in which the digital orthosis model is adapted individually to the body part based on the patient data,
and a manufacturing device for manufacturing the orthosis based on the adapted orthosis model, wherein the patient data comprises geometric data for describing a geometry of the body part,
wherein the device for capturing patient data has:
a holding device (1) for holding the body part in an adjustable correction position, a scanner for scanning the geometry of the body part held by the holding device (1) and generating the geometric data and a storage device for storing the patient data,
wherein the holding device
has a plurality of holding regions (3a-c) and one or more correction-force setting devices (5a-c, 5a'-c'), wherein at least one of the correction-force setting devices (5a-c, 5a'-c') is connected to at least one of the holding regions (1), and by means of the correction-force setting devices (5a-c, 5a'-c') correction forces can be exerted on the body part by way of the holding regions (3a-c) connected to the one or more correction-force setting devices (5a-c, 5a'-c'), and as a result the body part can be brought into the correction position,
and wherein each holding region (3a-c) has one, two or more holding elements (4a-c, 4a'-c') for fixing the body part in each case in at least one direction in space and/or for transmitting the correction forces exerted by means of the correction-force setting devices (5a-c, 5a'-c') to the body part,
wherein the method has the following steps:
i) the body part is held in the holding device in a set correction position,
ii) the geometry of the body part held by the holding device (1) is scanned by the scanner,
iii) the geometric data of the body part held in the holding device are generated and stored as patient data in the storage device,
iv) a digital orthosis model of the orthosis for the body part is generated and is adapted individually to the body part based on the patient data, and
v) the orthosis is manufactured based on the adapted orthosis model.

2. A method according to the preceding claim, **characterised in that** the holding device (1) has one or more force sensors (11) for measuring the correction forces exerted on the body part and at least one of the force sensors (11) is integrated in one of the holding elements (4a-c, 4a'-c') or a plurality of the force sensors (11) are integrated in some of or all the holding elements (4a-c, 4a'-c').

3. A method according to the preceding claim, **characterised in that** the force sensors (1) comprise pressure sensors or pressure measurement instruments.

4. A method according to one of the preceding claims, **characterised in that** at least one of the correction-force setting devices (5a-c, 5a'-c') has/have an adjustable-length rod (73), a telescopic rod, a linear guide, a linear cylinder (50a), two limbs (51b, 52b) in each case, the angular position of which is adjustable, a multiarticular arm (63-67, 63'-67') and/or an adjustable scissor joint.

5. A method according to the preceding claim, **characterised in that** the at least one correction-force setting device (5a-c, 5a'-c') has a triarticular arm (63-67, 63'-67') which has a middle and two outer articulations, wherein the middle articulation is a hinge joint (65, 65') and the outer articulations are ball-and-socket joints (63, 63', 64, 64'), and wherein at least one of the outer articulations is connected to one of the holding regions (3a-c), and correction forces can be exerted by varying a position of the outer and the middle articulation.

6. A method according to one of the preceding claims, **characterised in that** two holding regions (3a-c) are connected together by way of a correction-force setting device (5a-c, 5a'-c'), by means of which a correction force can be exerted on a first holding region (3a-c) relative to a second holding region (3a-c).

7. A method according to one of Claims 1 to 5, **characterised in that** the holding device (1) has a base frame (8) which defines a receiving region for the body part and to which are fastened the correction-force setting devices (5a-c, 5a'-c') which are supported on the base frame (8) and are suitable for exerting correction forces on the body part relative to the base frame (8) by way of the holding regions (3a-c) which are connected to the correction-force setting devices (5a-c, 5a'-c').

8. A method according to the preceding claim, **characterised in that** the base frame (8) is a rail, a rod or a telescopic rod (8a-c), or the base frame comprises rails (9) running in parallel or at an angle to each other, which define the receiving region in a direction to be corrected and to which the correction-force setting devices (5a-c, 5a'-c') are fastened, wherein a common cross-section through the rails (9) perpendicularly to the longitudinal extent of the rails (9) forms a regular or irregular n-gon, and wherein rails (9) which are adjacent to each other are connected rigidly together by at least one connecting element (10, 10'), and wherein n ≥ 3.

9. A method according to one of the preceding two claims, **characterised in that** the correction-force setting devices (5a-c, 5a'-c') are fastened displaceably and/or rotatably relative to the base frame (8) and positions of the correction-force setting devices (5a-c, 5a'-c') on the rails (9) are adjustable, in particular by means of quick-action clamps (74), adjusting screws, perforated rails and/or latch elements.

10. A method according to one of the preceding two claims, **characterised in that** at least one of the correction-force setting devices (5a-c, 5a'-c') has an adjustable-length rod (73), in particular a threaded rod, and is fastened to the rails (9) such that a longitudinal axis of the adjustable-length rod (73) is oriented largely at right angles to the rails (9) and correction forces on the body part can be varied by varying the length of the rod (73).

11. A method according to one of the preceding claims, **characterised in that** the correction-force setting devices (5a-c, 5a'-c') can be locked by means of a quick-action clamp (74) or a quick-action closure (66, 66') to exert a particular correction force.

12. A method according to one of Claims 8 - 10, **characterised in that** the rails (9) and/or the holding elements (4a-c, 4a'-c') contain or consist of dimensionally stable materials, in particular metal or plastic, in particular thermoplastic, thermoset and/or elastomeric plastics materials or glass.

13. A method according to one of the preceding claims, **characterised in that** the patient data comprise force measurement data for describing the correction forces exerted by the correction-force setting devices (5a-c, 5a'-c, 5a'-c') on the body part.

## Revendications

1. Procédé de fabrication d'une orthèse pour la correction d'un défaut d'alignement d'une partie du corps, l'orthèse étant fabriquée au moyen d'un dispositif de fabrication de l'orthèse,
le dispositif de fabrication de l'orthèse comprenant :
un dispositif pour la saisie de données concernant le patient et un dispositif de traitement de données pour la création d'un modèle d'orthèse numérique et pour la création d'un modèle d'orthèse adapté de l'orthèse, dans lequel le modèle d'orthèse numérique est adapté individuellement à la partie du corps sur la base des données concernant le patient,
et un dispositif de fabrication pour la fabrication de l'orthèse sur la base du modèle d'orthèse adapté, les données concernant le patient comprenant des données géométriques décrivant une géométrie de la partie du corps,
le dispositif de saisie de données concernant le patient concernant :
un dispositif de maintien (1) pour le maintien de la partie du corps dans une position de correction réglable, un appareil de scannage pour le scannage de la géométrie de la partie du corps maintenue par le dispositif de maintien (1) et la création des données géométriques, et un appareil d'enregistrement pour l'enregistrement des données concernant le patient,
le dispositif de maintien
comprenant une pluralité de zones de maintien (3a-c) et un ou plusieurs dispositifs de réglage de force de correction (5a-c, 5a'-c'), au moins un des dispositifs de réglage de force de correction (5a-c, 5a'-c') étant relié avec au moins une des zones de maintien (1) et les dispositifs de réglage de force de correction (5a-c, 5a'-c') permettant d'exercer, sur la partie du corps, des forces de correction par l'intermédiaire des zones de maintien (3a-c) reliées avec l'un ou plusieurs dispositifs de réglage de force de correction (5a-c, 5a'-c') et ainsi d'amener la partie du corps dans la position de correction,
et chaque zone de maintien (3a-c) comprenant deux éléments de maintien (4a-c, 4a'-c') pour la fixation de la partie du corps dans au moins une direction spatiale et/ou pour la transmission des forces de correction exercées au moyen des dispositifs de réglage de force de correction (5a-c, 5a'-c') à la partie du corps,
ce procédé comprenant les étapes suivantes :
i) la partie du corps est maintenue dans une position de correction réglée dans le dispositif de maintien,
ii) la géométrie de la partie du corps maintenue par le dispositif de maintien (1) est scanné par l'appareil de scannage,
iii) les données géométriques de la partie du corps maintenue dans le dispositif de maintien sont générées et enregistrées dans l'appareil d'enregistrement sous la forme de données concernant le patient,
iv) un modèle d'orthèse numérique de l'orthèse pour la partie du corps est créé et adapté individuellement à la partie du corps sur la base des données concernant le patient et
v) l'orthèse est fabriquée sur la base du modèle d'orthèse adapté.

2. Procédé selon la revendication précédente, **caractérisé en ce que** le dispositif de maintien (1) comprend un ou plusieurs capteurs de force (11) pour la mesure des forces de correction exercées sur la partie du corps et au moins un des capteurs (11) est intégré dans un des éléments de maintien (4a-c, 4a'-c') ou une pluralité de capteurs de force (11) sont intégrés dans certains ou dans tous les éléments de maintien (4a-c, 4a'-c').

3. Procédé selon la revendication précédente, **caractérisé en ce que** les capteurs de force (1) comprennent des capteurs de pression ou des appareils de mesure de pression.

4. Procédé selon l'une des revendications précédentes, **caractérisé en ce qu'**au moins un des dispositifs de réglage de la force de correction (5a-c, 5a'-c') comprend une tige réglable en longueur (73), une tige télescopique, un guidage linéaire, un vérin linéaire (50a), respectivement deux branches (51b, 52b) dont la position angulaire est ajustable, un bras à articulations multiples (63-67, 63'-67') et/ou une articulation à ciseaux ajustable.

5. Procédé selon la revendication précédente, **caractérisé en ce que** l'au moins un dispositif de réglage de la force de correction (5a-c, 5a'-c') comprend un bras à triple articulation (63-37),63'.67'), qui comprend une articulation centrale et deux articulations externes, l'articulation centrale étant une articulation de charnière (65, 65') et les articulations externes étant des articulations à rotules (63, 63', 64, 64') et au moins une des articulations externes étant reliée avec une des zones de maintien (3a-c) et la variation d'une position des articulations externes et de l'articulation centrale permettant d'exercer des forces de correction.

6. Procédé selon l'une des revendications précédentes, **caractérisé en ce que** deux zones de maintien (3a-c) sont reliées entre elles par l'intermédiaire d'un dispositif de réglage de la force de correction (5a-c, 5a'-c') au moyen duquel une force de correction peut être exercée sur une première zone de maintien (3a-c) par rapport à une deuxième zone de maintien (3a-c).

7. Procédé selon l'une des revendications 1 à 5, **caractérisé en ce que** le dispositif de maintien (1) comprend un châssis de base (8) qui définit une zone de logement pour la partie du corps et auquel sont fixés les dispositifs de réglage de la force de correction (5a-c, 5a'-c'), qui s'appuient contre le châssis de base (8) et qui sont conçus pour exercer des forces de correction par rapport au châssis de base (8) par l'intermédiaire de zones de maintien (3a-c) reliées avec les dispositifs de réglage de la force de correction (5a-c, 5a'-c').

8. Procédé selon la revendication précédente, **caractérisé en ce que** le châssis de base (8) est un rail, une toge ou une tige télescopique (8a-c) ou le châssis de base comprend des rails (9) parallèles ou formant un angle entre eux, qui définissent, dans une direction à corriger, la zone de logement et auxquels sont fixés les dispositifs de réglage de la force de correction (5a-c, 5a'-c'), une section transversale commune à travers les rails (9) perpendiculairement à l'extension longitudinale des rails (9) formant un polygone à n côtés régulier ou irrégulier et des rails (9) adjacents entre eux étant reliés de manière rigide entre eux par au moins un élément de liaison (10, 10') et moyennant quoi n ≥ 3.

9. Procédé selon l'une des deux revendications précédentes, **caractérisé en ce que** les dispositifs de réglage de la force de correction (5a-c, 5a'-c') sont fixés de manière coulissante et/ou rotative par rapport au châssis de base (8) et les positions des dispositifs de réglage de la force de correction (5a-c, 5a'-c') sur les rails (9) sont réglables, plus particulièrement au moyen de dispositifs de serrage rapide (74), de vis de réglage, de rails perforés et/ou d'éléments d'encliquetage.

10. Procédé selon l'une des deux revendications précédentes, **caractérisé en ce qu'**au moins un des dispositifs de réglage de la force de correction (5a-c, 5a'-c') comprend une tige réglable en longueur (73), plus particulièrement une tige filetée et est fixé aux rails (9) de façon à ce qu'un axe longitudinal de la tige réglable en longueur (73) soit orientée globalement perpendiculaire aux rails (9) et une variation de la longueur de la tige (73) permettant de faire varier les forces de correction sur la partie du corps.

11. Procédé selon l'une des revendications précédentes, **caractérisé en ce que** les dispositifs de réglage de la force de correction (5a-c, 5a'-c') peuvent être bloqués au moyen d'un dispositif de serrage rapide (74) ou d'une fermeture rapide (66, 66') pour l'application d'une force de correction déterminée.

12. Procédé selon l'une des revendications 8 à 10, **caractérisé en ce que** les rails (9) et/ou les éléments de maintien (4a-c, 4a'-c') comprennent ou sont constitués de matériaux rigides, plus particulièrement du métal ou une matière plastique, plus particulièrement des matières plastiques thermoplastiques, thermodurcissables ou élastomères ou du verre.

13. Procédé selon l'une des revendications précédentes, **caractérisé en ce que** les données concernant le patient comprennent des données de mesure de force pour la description des forces de correction exercées sur la partie du corps par les dispositifs de réglage de la force de correction (5a-c, 5a' -c, 5a'-c').
